# EUROPEAN PATENT SPECIFICATION

(11) **EP 3 025 656 B1**
(45) Date of publication and mention of the grant of the patent: **21.02.2018**
(21) Application number: 15201866.9
(22) Date of filing: 10.10.2008
(51) Int. Cl.: A61F 2/00, A61N 1/36, A61F 2/04, A61B 5/03, A61F 5/445

(54) **SYSTEM FOR TREATING A PATIENT HAVING AN INTESTINAL DISORDER**
SYSTEM ZUR BEHANDLUNG VON PATIENTEN MIT EINER DARMERKRANKUNG
SYSTÈME PERMETTANT DE TRAITER UN PATIENT ATTEINT DE TROUBLES INTESTINAUX

(30) Priority: 11.10.2007 US 960715 P; 11.10.2007 US 960716 P; 12.10.2007 US 960766 P
(43) Date of publication of application: 01.06.2016
(62) Divisional of application: 08838251.0
(73) Proprietor: Kirk Promotion LTD., 114 79 Stockholm (SE)
(72) Inventor: FORSELL, Peter, 6300 Zug (CH)
(74) Representative: Klunker IP Patentanwälte PartG mbB

(56) References cited:
- WO-A-02/39959
- US-A1- 2003 144 648
- US-A1- 2004 122 526
- US-A1- 2004 220 516
- US-B1- 6 752 754

## Description

### Background of the invention

The present invention relates to a system for treating a patient having a disorder related to the patient's intestine. Such disorder may be caused by injury, birth defect, cancer or other diseases, such as constipation or incontinence.

In an attempt to overcome such disorders, many different solutions have been proposed. These solutions often include surgery, in particular where a portion of the intestine has to be removed. The reason for such operation may be colorectal cancer, perforated diverticulitis or other kinds of diseases, such as ulceros colitis or Crohns disease. For instance, in the case of ileostomy, jejunostomy, colostomy and rectostomy operations the small intestine (jejunum or ileum) or the large intestine (colon or rectum) is cut and the open end of the healthy portion of the intestine is reattached either to a surgically created stoma in the patient's abdominal wall or, where possible, to the patient's rectum or anus or to tissue adjacent the patient's anus.

The problem then arises to control the intestinal contents flow and, more particularly, to prevent feces from exiting the patient's body uncontrolled. The patient is typically required to excrete into a colostomy bag. This is obviously inconvenient and, in addition, may cause skin irritation since such a bag arrangement requires an adhesive plate to be attached to the patient's skin in order to render the bag liquid tight.

US patent no. 4,222,377 suggests the use of an inflatable artificial sphincter comprising a cuff around the anal or urethral canal. A manually operated pump is implanted in the patient's scrotum for inflating and deflating the artificial sphincter.

Similarly, US patent no. 5,593,443 discloses an artificial hydraulic anal sphincter under voluntary control. More specifically, the patient may actuate a mechanical or electrical pump for inflating and deflating a cuff. The cuff consists of two parts positioned on opposite sides of the intestine and pressing the intestinal walls together when inflated.

US 6,752,754 B1 discloses an artificial rectum for replacing a portion of a patient's rectum. An inlet of the artificial rectum is operatively connected to the distal end of the patient's large intestine and communicates fecal matter to a macerator-type pump that discharges the feces through an outlet of the artificial rectum connected to the patient's anus. The pump includes a helical screw-type impeller, which when rotated creates shearing effects on the feces, causing it to move down the thread of the screw impeller and discharge through the patient's anus. US 2004/0122526 A1 discloses an implantable stomach prosthesis for surgically replacing or augmenting all or part of the antrum and/or pylorus of a stomach. The prosthesis controls the passage of food from the stomach to the small intestine. The prosthesis may be configured to chum ingested material and release it from the stomach through a prosthetic pyloric valve. The prosthesis includes an outer support structure, a flexible inner member forming a conduit for the movement of material, and at least one expandable member located between the outer support structure and inner member and for controlling the passage of food and/or to mimic the churning action of a patient's stomach. An implantable pump system is provided for inflating and deflating the expandable member(s).

### Summary of the invention

It is an object of the present invention to provide an improved system and method for treating a patient having a disorder related to the patient's intestine. Where in the following the word invention is used and/or features are presented as optional, this should be interpreted in such a way that protection is sought for the invention as claimed.

### INTERNAL RESERVOIR WITH IMPLANTABLE FLOW CONTROL DEVICE

A system according to the invention for treating a patient having a disorder related to the patient's intestine comprises a reservoir adapted for receiving and temporarily collecting therein intestinal contents and formed by at least one bent portion of human intestine, with laterally adjacent sections thereof being cut open along their mutual contact line and the resulting upper halves and lower halves thereof being interconnected so as to form the reservoir. The reservoir is further adapted to remain within the patient's body when emptying the reservoir. The system further comprises an artificial flow control device implantable in the patient's body and adapted to control flow of the intestinal contents from the reservoir when implanted in relation thereto, wherein the flow control device comprises a pump for emptying the reservoir and wherein the pump and the reservoir are separate from each other and wherein the pump is adapted to being implanted in the patient's body separate from but in close proximity to the reservoir so as to act on the reservoir from the outside thereof.

The internal reservoir will substantially extend the time period before the patient feels a need to excrete feces. Rather than using an external bag for that purpose which has to be attached, removed and cleaned, the internal reservoir remains within the patient's body. By combining such internal reservoir with an appropriate flow control device for emptying the reservoir, the patient's living circumstances are substantially improved.

### RESERVOIR FORMED FROM INTESTINE OR HUMAN TISSUE

According to the invention, the reservoir is formed from at least one bent portion of the patient's intestine. More specifically, laterally adjacent sections of the intestine are cut open along their mutual contact line and the resulting upper halves and lower halves thereof are then interconnected so as to form the reservoir. The interconnection can advantageously be made with staplers, possibly including bonding with a biocompatible glue, but sewing is likewise an option. Alternatively, the reservoir may also be formed from surgically modified and connected human tissue.

### ISOLATED LATERAL ARRANGEMENT OF RESERVOIR

The reservoir may be laterally connected to the intestine by means of a single passage through which the intestinal contents are fed in both ways, i.e. from the intestine into the reservoir and, when the reservoir is emptied, in the reverse direction from the reservoir back to the intestine and further out of the body. Accordingly, the system according to the invention comprises a passage in flow communication with the reservoir and adapted to being connected to a surgically created lateral opening in a wall of the patient's intestine, said passage being arranged for transferring intestinal contents to and from the reservoir.

### THROUGH-FLOW ARRANGEMENT OF RESERVOIR

Instead of a single passage, two passage may be provided, a first passage leading feces to the reservoir and a second passage different from the first passage leading the feces from the reservoir when the reservoir is emptied.

Accordingly the system of the present invention may comprise a first passage in flow communication with the reservoir and adapted to being connected to a surgically created first opening of the patient's intestine, said first passage being arranged for transferring intestinal contents to the reservoir, and further comprising a second passage in flow communication with the reservoir, said second passage being arranged for transferring intestinal contents from the reservoir.

The second passage must not necessarily redirect the feces back to the intestine, it may as well be adapted to being surgically connected to a surgically created stoma or to the patient's rectum or anus or to tissue adjacent the patient's anus.

On the other hand, rather than directly connecting the second passage to the stoma or anus, the second open end portion may be adapted to being connected to a healthy portion of the patient's small intestine or of the patient's large intestine. Since the small intestine and large intestine have different diameters and wall thickness, the structure of the second open end portion can be substantially different in these cases. The healthy portion of the intestine may then be connected to the patient's rectum or anus or to tissue adjacent the patient's anus or to use that portion for creating a stomy.

### LATERAL ATTACHMENT

In particular, the second passage may be adapted to being surgically connected to a second surgically created opening of the patient's intestine, this not being restricted to a connection to a cross-sectional opening. More specifically, according to a preferred embodiment, the second passage is adapted to being connected to a lateral opening in the wall of the patient's intestine. Similarly, it is preferred that also the first passage is adapted to being connected to a lateral opening in the wall of the patient's intestine. Lateral attachment of the passage or passages between the intestine and the reservoir has the advantage that the forces resulting from the peristaltic waves moving along the intestine have less impact on the connection. In frontal connections, i.e. where the reservoir is attached to a cross-sectional opening of the intestine, the peristaltic waves tend to pull the intestine away from the connection, this requiring special securing measures.

In order to connect the reservoir to the intestine, the first passage is preferably adapted to being bonded and/or sewn and or stapled to the intestine, this applying to both a cross-sectional and a lateral attachment.

### VALVE AS PART OF THE FLOW CONTROL DEVICE

As a main element of the flow control device for controlling flow from the reservoir, in particular for emptying the reservoir, there may be provided one or more valve and/or pump.

### EXIT VALVE

For instance, the at least one valve may include an exit valve preventing intestinal contents flow from the reservoir in its closed position. Preferably, the exit valve is a normally closed valve so that no energy is needed to keep the valve closed during the system's inactive periods.

### ENTRY VALVE IN ADDITION TO EXIT VALVE

In addition, the flow control device may comprise an entry valve allowing intestinal contents to flow towards the reservoir in its open position. This can be advantageous particularly during the emptying of the reservoir, when the entry valve should be closed. Therefore, the entry valve is preferably a normally open valve. Accordingly, the exit valve and the entry valve are preferably adapted to cooperate such that when one of the two valves is closed, the respective other valve is open, and vice versa.

### VALVE ARRANGEMENT (INTERNAL, INTERSECTING, OUTSIDE)

The valves can be arranged in many different ways, depending on the type of valve. For instance, at least one of the valve or valves, respectively, can be adapted to being permanently implanted inside the patient's intestine. Or, where at least one of the valve or valves, respectively, has an upstream open end and has a downstream open end in fluid connection with the upstream open end, the upstream open end can be adapted to being connected to a surgically created opening of the patient's intestine and the downstream open end can be adapted to being connected to either one of a surgically created opening of the patient's intestine, a surgically created stoma, the patient's anus or tissue adjacent the patient's anus. Alternatively, at least one of the valve or valves, respectively, may be adapted to being implanted inside the patient's body outside a section of the patient's intestine and may comprise at least one element adapted to act on the intestine section from the outside thereof so as to act on and, in particular, prevent intestinal contents flow through the intestine section. This latter valve arrangement is advantageous inasmuch its installment does not require any surgery on the respective part of the intestine.

### VALVE TYPES

As regards the various valve types that may be employed, the at least one valve may e.g. comprise a central opening which is normally closed by resilient means that can be urged apart mechanically by inserting a conduit through the central opening so as to open the central opening of the valve. In the simplest embodiment, the valve may be opened by mechanical force, such as by inserting a tube from outside the patient's body through the valve. The valve in this case can be a simple non-return valve.

According to a more complex embodiment, the at least one valve may comprise a compartment with a variable volume adapted to open and close the valve by changing the compartment's volume. Advantageously, the at least one valve comprises at least one passage for filling and emptying the compartment with hydraulic fluid. The compartment preferably has at least one flexible wall defining an opening for the intestine or a conduit of the reservoir to pass through, the opening being adapted to close upon increase of the compartment's volume.

According to a different embodiment, the at least one valve may be a flap valve permanently implanted inside the patient's intestine. The flap valve may for instance comprise a rotatable disc.

According to a very specific embodiment, where the at least one valve is implanted inside the patient's body outside a section of the patient's intestine to act on the intestine section from the outside thereof, the valve may comprise at least one electrical stimulation device adapted to electrically stimulate muscle or neural tissue of an intestine section so as to cause at least partial contraction of the intestine section. This is a very gender way of constricting the intestine. The stimulation device preferably comprises at least one electrode adapted to apply electric pulses to the intestine section.

It is particularly advantageous to make use of a stimulation device which is adapted to stimulate different portions of the intestine section over time. Thus, different portions of the intestine section can be constricted by stimulation at different times in any predetermined stimulation pattern, thereby giving the intestine portions currently not stimulated time to recover and, thus, improving the blood circulation in the respective intestine section.

Furthermore, the stimulation device can specifically be adapted to stimulate, over time, the different portions of the intestine section in a wave like manner in a direction opposite to natural intestinal contents flow. As a result, the valve counteracts the natural intestinal contents flow, thereby improving the valve's closing function.

Alternatively, or preferably in addition to the stimulation device, the at least one valve may comprise a constriction device implanted in the patient's body for at least partly constricting the intestine section mechanically from outside the intestine section. Where the stimulation device is combined with the constriction device, the stimulation device and the constriction device preferably act on the same intestine section. In that case, it is advantageous if the constriction device in its normal condition constricts the intestine section only partly, in order not to damage the intestine over time. Complete constriction and, thus, closing of the intestine may then be obtained by additionally stimulating the intestine section in a manner as described before.

In addition, when constriction of the intestine section caused by the constriction device is released, the stimulation device may, if accordingly adapted, be used to pump intestinal contents along the intestine section by, over time, stimulating different portions of the intestine section in a wave like manner in a direction of natural intestinal contents flow. In this situation, the valve may incorporate the additional function of a pump for actively supporting the discharge of feces from the human body.

### PUMP AS PART OF THE IMPLANTABLE FLOW CONTROL DEVICE

According to the invention, the flow control device comprises the pump for emptying the reservoir, a variety of different types of pumps may be employed.

For instance, the pump may be adapted for emptying the reservoir by squeezing the reservoir, if the reservoir has a flexible wall that allows for squeezing.

According to the invention, the pump and the reservoir are separate from each other and the pump is adapted to being implanted in the patient's body separate from but in close proximity to the reservoir so as to act on the reservoir from the outside thereof. For instance, the reservoir may have a flexible wall and the pump may comprise a movable piston, with a front end of the piston adapted to act on the flexible wall of the reservoir from the outside thereof upon advancement of the piston.

As an example not forming part of the invention, the pump and the reservoir can be fixedly connected to one another. For instance, the reservoir may be formed by a bellow, said bellow having an end wall closing the bellow at one end thereof and said end wall making part of the pump such that a volume of the bellow is reduced upon advancement of said end wall. Preferably, the bellow is made of a resilient material so as to urge the bellow into a normally expanded position.

In another example where the pump and the reservoir are fixedly connected to one another, the pump comprises a movable piston with a front end of the piston extending into the reservoir such that a volume of the reservoir can be reduced upon advancement of the piston. Preferably, the pump and the reservoir are comprised in a common housing so that they can be implanted as a unit. Furthermore, it is advantageous if the piston is spring loaded so as to urge the piston into a normally retracted position.

As a further example, the pump may be adapted for being permanently arranged inside the reservoir.

As a still further alternative, the pump and the reservoir can be provided separate from each other with the pump being implanted inside the patient's body outside the patient's intestine. Similarly to the very specific valve embodiment described above, according to this alternative the pump comprises at least one electrical stimulation device adapted to electrically stimulate muscle or neural tissue of an intestine section so as to cause at least partial contraction of the intestine section. Again, the stimulation device preferably comprises at least one electrode adapted to apply electric pulses to the intestine section. Furthermore, the stimulation device may be adapted to stimulate different portions of the intestine section over time so as to pump intestinal contents along the intestine section by, over time, stimulating the different portions of the intestine section in a wave like manner in the direction of natural intestinal contents flow.

Also, the pump may comprise a constriction device similar to the valve embodiment described earlier for at least partly constricting the intestine section mechanically, whereby the constriction device is preferably adapted to pump intestinal contents along the intestine section by, over time, constricting different portions of an intestine section in a wave like manner in the direction of natural intestinal contents flow.

Again, the stimulation device may be combined with the constriction device so as to pump the intestinal contents along the intestine section by, over time, stimulating the different portions of the intestine section in a wave like manner in a direction of natural intestinal contents flow, when constriction of the intestine section caused by the constriction device is released at the respective portions.

Finally, the pump may also be constituted by a manually drivable pump and may comprise an actuator for manually driving the pump. The actuator in this case is preferably arranged for subcutaneous implantation so as to be operable from outside the patient's body.

### MOTOR

Where the valves or pump or any other element of the flow control device is not or not only manually drivable, at least one motor can be provided for automatically driving one or more of the elements of the flow control device. The motor is preferably arranged to be driven by electric or electromagnetic energy.

A motor in the sense of the present invention is a device that transforms energy other than mechanical energy into mechanical energy. While a pump in the sense of the present invention is a device for advancing liquid or pasty material, a pump may at the same time be a motor in certain circumstances, such as where the transformation of energy into mechanical energy causes advancement of the liquid or pasty material without any intervening mechanical means such as a piston, bellow or the like.

For instance, the at least one motor can be arranged for driving at least one of the valve or valves, respectively, between its closed and open position. Also, the at least one motor can be arranged for driving the pump.

A manually operable switch may be provided for activating the at least one motor, the switch being preferably arranged for subcutaneous implantation so as to be operable from outside the patient's body.

### ENERGY SOURCE

In a preferred embodiment, an energy source is provided for supplying energy directly or indirectly to at least one energy consuming part of the system. Preferably, the energy source includes a battery or an accumulator, such as one or more of a rechargeable battery and a capacitor, as an energy storage means. The energy storage means is advantageously adapted for being implanted inside the patient's body.

### WIRELESS ENERGY TRANSMISSION

Energy is preferably transmitted wirelessly. Thus, where the energy source is provided for supplying energy directly or indirectly to at least one energy consuming part of the system, the energy source may comprise a wireless energy transmitter adapted to wirelessly transmit energy from outside the patient's body to the at least one energy consuming part. Alternatively, where the energy source includes a battery or an accumulator, in particular one which is implanted in the patient's body, the energy source may comprise a wireless energy transmitter adapted to wirelessly transmit energy from outside the patient's body to the energy storage means.

### ENERGY TRANSMISSION FEEDBACK

A feedback subsystem, which can make part of a control device described subsequently, can advantageously be provided to wirelessly send feedback information related to the energy to be stored in the accumulator from inside the human body to the outside thereof. The feedback information is then used for adjusting the amount of wireless energy transmitted by the energy transmitter. Such feedback information may relate to an energy balance which is defined as the balance between an amount of wireless energy received inside the human body and an amount of energy consumed by the at least one energy consuming part. Alternatively, the feedback information may relate to an energy balance which is defined as the balance between a rate of wireless energy received inside the human body and a rate of energy consumed by the at least one energy consuming part.

Also, the transmission of energy from the energy storage means to the at least one energy consuming part may be performed wirelessly by means of an accordingly adapted wireless energy transmitter.

Preferably, in order to reduce the number of parts and possibly increase the system's efficiency, the energy consuming part can be adapted to directly transform the wirelessly transmitted energy into kinetic energy. Otherwise, it will be necessary to provide an implantable energy transforming device for transforming the wireless energy, preferably into electric energy. In this case, it is further preferred to set up the system such that the energy consuming part is driven with the electric energy, as said energy transforming device transforms the wireless energy into the electric energy.

The energy transmitter can be adapted to generate an electromagnetic field, a magnetic field or an electrical field. The wireless energy may be transmitted by the energy transmission device by at least one wireless signal. More specifically, the energy transmitter may be adapted to transmit the energy by at least one wireless energy signal, which may comprise an electromagnetic wave signal, including at least one of an infrared light signal, a visible light signal, an ultra violet light signal, a laser signal, a microwave signal, an X-ray radiation signal, and a gamma radiation signal. Also, the wireless energy signal may comprise a sound or ultrasound wave signal. Furthermore, the wireless energy signal may comprise a digital or analog signal or a combination thereof.

### GALVANIC ENERGY TRANSMISSION

Where energy is not transmitted wirelessly, galvanic coupling elements should be provided at least between the energy source and the motor for transmitting energy to the motor in contacting fashion.

### CONTROL UNIT

It is advantageous to provide a control unit adapted to directly or indirectly control one or more elements of the system, such as for controlling opening of the exit valve and/or closing of the entry valve, in particular in a manner such that when one of the two valves is closed, the respective other valve is open, and vice versa. The control unit can also be adapted to control actuation of the pump.

The control unit is preferably operable by the patient, e.g. particularly in order to empty the reservoir.

At least part of the control unit may be adapted to be implantable in the patient's body. For instance, a manually operable switch may be provided for activating the control unit, the switch preferably being arranged for subcutaneous implantation so as to be operable from outside the patient's body. Also, the control unit may comprise a first part adapted for implantation in the patient's body and a second part adapted to cooperate with the first part from outside the patient's body. In this case, the control unit can be adapted to transmit data from the external second part of the control unit to the implanted first part of the control unit in the same manner as energy is transmitted to the at least one energy consuming part.

That is, the second part of the control unit may be adapted to wirelessly transmit a control signal to the implantable first part of the control unit for controlling the at least one energy consuming part from outside the patient's body. Also, the implantable first part of the control unit may be programmable via the second part of the control unit. Furthermore, the implantable first part of the control unit may be adapted to transmit a feedback signal to the second part of the control unit.

### SENSOR

Furthermore, a physical parameter sensor adapted to directly or indirectly sense a physical parameter of the patient can be provided. The physical parameter sensor may be adapted to sense at least one of the following physical parameters of the patient: a pressure within the reservoir, a pressure within the patient's intestine, an expansion of the reservoir, a distension of an intestinal wall of the patient's intestine, a movement of the intestinal wall.

Similarly, a functional parameter sensor adapted to directly or indirectly sense a functional parameter of the system can be provided, wherein the functional parameter sensor may be adapted to sense at least one of the following functional parameters of the system: a pressure against a part of the system such as the reservoir, a distension of a part of the system such as a wall of the reservoir, an electrical parameter such as voltage, current or energy balance, a position or movement of a movable part of the system.

Preferably, an indicator is coupled to the sensor or sensors, the indicator being adapted to provide a signal when a sensor senses a value for the parameter beyond a predetermined threshold value. The sensor signal may comprise at least one of the following types of signals: a sound signal, a visual signal.

### INTESTINAL CONTENTS COLLECTING DEVICE (WITH "EXTERNAL" PUMP)

As mentioned before, in the simplest embodiment, an intestinal contents collecting device may used to be temporarily applied from outside the patient's body when the reservoir is to be emptied. According to a preferred embodiment, the collecting device may comprise a front open end adapted to be applied towards the exit valve so as to provide a flow passage from the exit valve towards the collecting device. More specifically, the collecting device front open end is preferably adapted to be applied to the exit valve so as to open the valve and thereby provide said flow passage towards the collecting device. Where the exit valve is normally closed by resilient means, said front open end is adapted to be inserted through the central opening of the exit valve so as to urge apart the resilient means normally closing the central opening.

The collecting device preferably comprises a suction pump, which may comprise a piston-cylinder-arrangement. The suction pump may be adapted to be driven manually, in particular where it is intended for use as a back-up pump for a situation where the pump of the flow control device is out of operation. However, preferably a motor is connected to the suction pump for driving the pump automatically.

### METHOD OF TREATMENT (IMPLANTATION)

The description does not only relate to the system described above, but also to a method of treating a patient having a disorder related to the patient's intestine.

### RESERVOIR FORMED FROM INTESTINE

As mentioned before, the reservoir of the system may be made from the patient's intestine. A respective surgical method of treating the patient would comprise the steps of:
- cutting the patient's skin and abdominal wall,
- dissecting an area of the patient's intestine,
- cutting the patient's intestine along a mutual contact line of laterally adjacent sections of a bent portion thereof and connecting by suturing and/or stapeling the resulting upper and lower halves of the intestine so as to form a reservoir,
- implanting a flow control device so as to permanently reside inside the patient's body and adapted to control flow of intestinal contents from the reservoir to outside the patient's body, and
- thereafter, permanently closing the abdominal wall and skin.

A respective laparoscopic surgical method of treating the patient would comprise the steps of:
- making a small opening in the patient's skin and abdominal wall,
- introducing a needle in the abdominal cavity,
- inflating the abdominal cavity with gas,
- inserting at least one trocar into the cavity,
- introducing a camera through the trocar,
- inserting at least one dissecting instrument preferably through a second trocar,
- dissecting an area of the intestine,
- cutting the patient's intestine along a mutual contact line of laterally adjacent sections of a bent portion thereof and connecting by suturing and/or stapling the resulting upper and lower halves of the intestine so as to form a reservoir,
- implanting a flow control device so as to permanently reside inside the patient's body and adapted to control flow of intestinal contents from the reservoir to outside the patient's body,
- extracting the instruments, camera and trocar, and in relation thereto
- suturing, if necessary, the abdominal wall and permanently closing the skin.

As also mentioned before, the system may be surgically connected to a surgically created stoma or to the patient's rectum or anus or to tissue adjacent the patient's anus. This would require, where a stomy is involved, the following additional steps:
- cutting the patient's skin and abdominal wall so as to create an opening for an intestinal stomy,
- dissecting the area of the opening,
- dividing the intestine downstream of the reservoir so as to maintain an upstream natural intestine section still connected to the reservoir with a cross-sectional opening at the downstream end thereof,
- dissecting the mesentery of the upstream natural intestine section in the area of the cross-sectional opening at the downstream end thereof to prepare for creating the intestinal stomy,
- advancing the upstream natural intestine section through the abdominal wall and skin and
- suturing the upstream natural intestine section in the area of the cross-sectional opening to the skin with the intestinal mucosa turned inside out, thereby achieving the intestinal stomy.

Where the system may be surgically connected to a the patient's anus or to tissue adjacent the patient's anus, this would require the following additional steps:
- dividing the intestine so as to create an upstream natural intestine section having a cross-sectional opening at the downstream end thereof and a downstream natural intestine section leading to the patient's anus,
- dissecting the area of the patient's anus and surgically separating the downstream natural intestine section from the patient's anus, whereas the steps of dividing the intestine and separating the intestine section leading to the patient's anus can alternatively be carried out in reversed order,
- dissecting the mesentery of the upstream natural intestine section in the area of the cross-sectional opening at the downstream end thereof to prepare for connecting the upstream natural intestine section to the patient's anus or tissue adjacent the patient's anus,
- advancing the downstream end of the upstream natural intestine section through the patient's anus, and
- suturing the cross-sectional opening of the upstream natural intestine section to the patient's anus or tissue adjacent the patient's anus.

In line with the very specific valve embodiment described previously, the method may further involve the step of implanting at least one electrical stimulation device in the vicinity of an intestine section so as to allow for at least partial contraction of the intestine section by means of electrical stimulation of muscle or neural tissue with the aid of the electrical stimulation device. Preferably, electric pulses are applied to the intestine section by means of the stimulation device.

Preferably the stimulation device is implanted along the intestine section so as to be able to stimulate different portions of the intestine section over time. More specifically, the stimulation device may be implanted to pump intestinal contents along the intestine section by, over time, stimulating the different portions of the intestine section in a wave like manner.

Also, a constriction device may be implanted so as to allow for at least partial mechanical constriction of the intestine section by means of the constriction device. The constriction device may advantageously combined with the stimulation device so as to allow for adding further constriction of the intestine section by stimulating the intestine section with stimulation pulses. In particular, this may be used for pumping intestinal contents along the intestine section by, over time, stimulating the different portions of the intestine section in a wave like manner, when constriction of the intestine section caused by the constriction device is released.

### ARTIFICIAL RESERVOIR

While the reservoir may be made from the patient's intestine in the manner as described earlier, an artificial reservoir made from non-biologic material may as well be used. In this case, a surgical method of treating a patient may comprise the steps of:
- cutting the patient's skin and abdominal wall,
- dissecting an area of the patient's intestine,
- surgically creating an opening in the dissected intestinal area,
- affixing to the opening a reservoir so as to receive and temporarily collect therein intestinal contents from the patient's intestine,
- implanting a flow control device so as to permanently reside inside the patient's body and adapted to control flow of intestinal contents from the reservoir to outside the patient's body, and
- suturing the abdominal wall and skin.

A respective laparoscopic surgical method of treating a patient may comprise the steps of:
- making a small opening in the patient's skin and abdominal wall,
- introducing a needle in the abdominal cavity,
- inflating the abdominal cavity with gas,
- inserting at least one trocar into the cavity,
- introducing a camera through the trocar,
- inserting at least one dissecting instrument preferably through a second trocar,
- dissecting an area of the intestine,
- surgically creating an opening in the dissected intestinal area,
- affixing to the opening an artificial reservoir so as to receive and temporarily collect therein intestinal contents from the patient's intestine and
- implanting a flow control device so as to permanently reside inside the patient's body and be adapted to control flow of intestinal contents from the reservoir to outside the patient's body,
- connecting the intestine to the artificial reservoir with securing means,
- extracting the instruments, camera and trocar, and in relation thereto
- suturing, if necessary, the abdominal wall and permanently closing the skin.

In the afore described embodiments the reservoir is attached to an opening of the intestine, which opening may be a lateral opening created in the intestinal wall so as to allow intestinal content to flow to the reservoir and, possibly, also from the reservoir back to the intestine through the same opening. However, the opening may as well be a cross-sectional opening of the intestine, which can be obtained by dividing the intestine. In the latter case of a connecting the reservoir to a cross-sectional opening of the intestine, a respective surgical method of treating a patient would comprise the steps of:
- cutting the patient's skin and abdominal wall,
- dissecting an area of the patient's intestine,
- dissecting a portion of the dissected intestinal area such that intestinal mesentery connected thereto is opened in such a way that supply of blood through the mesentery to the dissected intestinal area is maintained as much as possible on both sides of the dissected portion,
- dividing the patient's intestine in the dissected portion so as to create an upstream part of the intestine with a first intestinal opening and a downstream part of the intestine with a second intestinal opening with the mesentery still maintaining a tissue connection between the upstream and downstream intestine parts,
- affixing to the first intestinal opening an artificial reservoir so as to receive and temporarily collect therein intestinal contents from the patient's intestine,
- affixing the reservoir to the second intestinal opening so as to allow for discharging intestinal contents from the reservoir through the second intestinal opening,
- implanting a flow control device so as to permanently reside inside the patient's body and be adapted to control flow of intestinal contents from the reservoir through the downstream intestine part, and
- suturing the abdominal wall and skin.

A corresponding laparascopic surgical method of treating a patient comprises the steps of:
- making a small opening in the patient's skin and abdominal wall,
- introducing a needle in the abdominal cavity,
- inflating the abdominal cavity with gas,
- inserting at least one trocar into the cavity,
- introducing a camera through the trocar,
- inserting at least one dissecting instrument preferable through at least a second trocar,
- dissecting an area of the intestine,
- dissecting a portion of the dissected intestinal area such that intestinal mesentery connected thereto is opened in such a way that supply of blood through the mesentery to the dissected intestinal area is maintained as much as possible on both sides of the dissected portion,
- dividing the patient's intestine in the dissected portion so as to create an upstream part of the intestine with a first intestinal opening and a downstream part of the intestine with a second intestinal opening with the mesentery still maintaining a tissue connection between the upstream and downstream intestine parts,
- affixing to the first intestinal opening an artificial reservoir so as to receive and temporarily collect therein intestinal contents from the patient's intestine,
- affixing the reservoir to the second intestinal opening so as to allow for discharging intestinal contents from the reservoir through the second intestinal opening,
- implanting a flow control device so as to permanently reside inside the patient's body and be adapted to control flow of intestinal contents from the reservoir to the downstream intestine part,
- connecting the intestine to the artificial reservoir with securing means,
- extracting the instruments, camera and trocar, and in relation thereto
- suturing, if necessary, the abdominal wall and permanently closing the skin.

Depending on where the intestine is divided, the "second" cross-sectional intestinal opening is created either in the patient's small intestine or in the patient's large intestine.

Where the downstream part of the intestine is to be attached to a surgically created stomy, this may involve the following additional steps:
- cutting the patient's skin and abdominal wall to create an opening for an intestinal stomy,
- dissecting the area of the opening,
- dividing the downstream intestine part so as to create at the downstream end of the downstream intestine part a third opening,
- dissecting the mesentery of the downstream intestine part in the area of the third opening to prepare for creating the intestinal stomy,
- advancing the downstream intestine part through the abdominal wall and skin and
- suturing the third opening to the skin with the intestinal mucosa turned inside out, thereby achieving the intestinal stomy.

Where the downstream part of the intestine is to be attached to the patient's anus or tissue adjacent the patient's anus, rather than to a surgically created stomy, this may involve the following additional steps:
- dividing the intestine so as to create an upstream natural intestine section having a cross-sectional opening at the downstream end thereof and a downstream natural intestine section leading to the patient's anus,
- dissecting the area of the patient's anus and surgically separating the downstream natural intestine section from the patient's anus, whereas the steps of dividing the intestine and separating the intestine section leading to the patient's anus can alternatively be carried out in reversed order,
- dissecting the mesentery of the upstream natural intestine section in the area of the cross-sectional opening at the downstream end thereof to prepare for connecting the upstream natural intestine section to the patient's anus or tissue adjacent the patient's anus,
- advancing the downstream end of the upstream natural intestine section through the patient's anus, and
- suturing the cross-sectional opening of the upstream natural intestine section to the patient's anus or tissue adjacent the patient's anus.

### EXIT VALVE

Where the flow control device comprises an exit valve preventing intestinal contents to flow through the valve in its closed position, the step of implanting the flow control device may comprise:
- cutting at least one portion of the patient's intestine so as to create at least one artificial opening in the intestine downstream of the reservoir,
- affixing the exit valve to the artificial intestinal opening, and
- connecting the exit valve to a pre-existing opening in the patient's body.

The step of connecting the exit valve to a pre-existing opening in the patient's body may comprise either one of affixing the exit valve to the patient's anus or tissue adjacent the patient's anus, to a portion of the patient's intestine leading to the patient's anus, or - after surgically creating an artificial stoma in the patient's abdominal wall and skin - to the surgically created stoma stoma. Alternatively, the exit valve may be connected to a portion of the patient's intestine and that portion of the patient's intestine may be used to create the stomy.

### LATERAL CONNECTION (OF RESERVOIR, PUMP OR VALVE) TO INTESTINE

It was described before how the reservoir may be attached between two cross-sectional openings of a divided intestine. It is, however, also possible to surgically create an opening in the dissected intestinal area by cutting the artificial intestinal opening into a lateral wall of the intestine so as to create a lateral artificial intestinal opening, and attach the reservoir to the lateral opening. In this case, the intestine is preferably permanently closed downstream of the lateral artificial intestinal opening.

### BY-PASS CONNECTION

Where two lateral artificial intestinal openings are cut at different locations of the intestine's lateral wall, the intestine may further be cut between the two locations and the cut ends permanently closed or the intestine may simply be permanently closed between the two locations, e.g. by suturing and/or stapling, wherein the reservoir is affixed in flow connection intermediate the two artificial intestinal openings.

### END-CONNECTION (OF RESERVOIR OR EXIT VALVE; SLEEVE/BULGE CONNECTOR)

Where the step of surgically creating an opening in the dissected intestinal area comprises cutting the artificial intestine completely in a crosswise direction so as to create at least one artificial opening in the intestine, the step of affixing to the opening may comprise:
- connecting a conduit to a section of the intestine by inserting an end of the conduit into the artificial intestinal opening, and
- placing a flexible sleeve so as to extend over the intestine and conduit such that at least part of the intestine is located intermediate the sleeve and the outer surface of the conduit.

Where the flexible sleeve is mounted on the outer surface of the conduit so as to be foldable upon itself, the step of placing the flexible sleeve so as to extend over the intestine and conduit may comprise folding the flexible sleeve upon itself such that at least part of the intestine is located intermediate the folded sleeve.

Alternatively, or in addition, where the step of surgically creating an opening in the dissected intestinal area comprises cutting the artificial intestine completely in a crosswise direction so as to create at least one artificial opening in the intestine, the step of affixing to the opening may comprise:
- connecting a conduit having a bulge formed on the outside thereof to a section of the intestine by inserting an end of the conduit into the artificial intestinal opening so that the intestine extends over the bulge from one side of the bulge, and
- advancing a blocking ring over the intestine towards the bulge from the respective other side of the bulge such that the intestine is located intermediate the conduit's outer surface and the blocking ring.

The afore-mentioned conduits with a sleeve or with a bulge serve to improve the strength of the connection against forces which result from the peristaltic movement of the intestine and tend to pull on the intestine. The conduit may also combine a sleeve and a bulge. The conduit may lead to or from the reservoir or may make part of the valve or pump.

### EXTERNAL/INTERNAL ARRANGEMENT OF EXIT VALVE

Where the flow control device comprises an exit valve preventing intestinal contents flow through the valve in its closed position, the step of implanting the flow control device may comprise either placing the exit valve outside and adjacent to a section of the intestine downstream of the reservoir so as to allow acting on said intestine section from the outside thereof by means of the exit valve, or affixing the exit valve within a section of the intestine downstream of the reservoir so as to allow closing said intestine section by means of the exit valve when the valve is in its closed position.

### BOND AND SUTURE CONNECTION

The step of affixing to the opening preferably comprises bonding, possibly further including suturing and/or stapling, an element to the intestine so as to surround and close the artificial intestinal opening.

### PUMP

Where the flow control device comprises a pump for emptying the reservoir, the step of implanting the flow control device may comprise implanting the pump in the patient's abdomen either separate from but in close proximity to the reservoir so that it can act on the reservoir from the outside thereof, or fixedly connected to the reservoir. Alternatively, the pump may be implanted in the reservoir so that it can act on the intestinal contents in the reservoir from the inside the reservoir.

### MANUAL DRIVE

Where the flow control device comprises an actuator for manually driving the pump, such actuator is preferably implanted subcutaneously so as to be operable from outside the patient's body.

### MOTOR/PUMP

As mentioned before, at least one motor and/or a pump may be implanted for automatically driving one or more elements of the flow control device.

### SWITCH

Where the motor and/or pump comprises a manually operable switch for activating the motor and/or pump, the switch is preferably implanted subcutaneously so as to be operable from outside the patient's body.

### ENERGY SOURCE / ENERGY TRANSMISSION

As mentioned before, an energy source, possibly comprising an energy storage means, may be implanted inside the patient's body for supplying at least one energy consuming part with energy.

Furthermore, an energy transforming device for transforming wireless energy into electric energy may also be implanted. Alternatively or in addition thereto, galvanic coupling elements may be implanted for transmitting energy to the energy consuming part in contacting fashion.

### CONTROL UNIT

Also, as mentioned before, at least a part of a control unit may be implanted to directly or indirectly control one or more elements of the flow control device.

Where the control unit comprises a manually operable switch for activating the control unit, the switch is preferably implanted subcutaneously so as to be operable from outside the patient's body.

### SENSOR

As mentioned before, one or more physical and/or functional parameter sensors may be implanted to directly or indirectly sense physical and/or functional parameters inside the patient and in the system implanted inside the patient. Where the sensor is a pressure sensor, it may be placed in the reservoir or intestine so as to sense the pressure within the reservoir or intestine, respectively. Where the sensor is a tension sensor, it may be placed in contact with the reservoir or intestine so as to sense an expansion of the reservoir or intestine, respectively. Where the sensor is a movement sensor, it may be placed in contact with the intestine so as to sense movement of the intestine. The functional sensor may be adapted to measure at least one of the following functional parameters of the system: an electrical parameter such as voltage, current or energy balance or a stimulation parameter in relation to the system.

### USE

Once the system according to the invention has been properly installed, the flow control device can be used for emptying the reservoir implanted in the patient.

### EXIT AND ENTRY VALVE

More specifically, where the flow control device comprises an exit valve preventing intestinal contents flow from the reservoir in its closed position, the method of use comprises the steps of opening the exit valve and then emptying the reservoir. Where in addition to the exit valve an entry valve is provided allowing intestinal contents to flow towards the reservoir in its open position, the method further comprises the step of closing the entry valve before emptying the reservoir.

According to one embodiment, the patient's intestine will be constricted by means of the exit valve so as to prevent intestinal contents flow through the constricted intestine section while the reservoir is not to be emptied. In addition, the patient's intestine may be constricted by means of the entry valve so as to prevent intestinal contents flow through the constricted intestine section while the reservoir is to be emptied.

As mentioned before, the step of constricting may comprise the step of electrically stimulating muscle or neural tissue of the intestine section to cause at least partial contraction of the intestine section, preferably by applying electric pulses to the intestine section. More preferably, different portions of the intestine section may be stimulated over time. This specifically allows for stimulating, over time, the different portions of the intestine section in a wave like manner in a direction opposite to natural intestinal contents flow, thereby supporting the closing function of the valve.

As mentioned before, constriction may also be achieved by mechanically constricting the intestine section at least partly. Where this is combined with electrically stimulation, it is preferred that the steps of mechanically constricting and electrically stimulating are performed on the same intestine section. More specifically, in order to empty the reservoir intestinal contents along the intestine section can be pumped by, over time, electrically stimulating different portions of the intestine section in a wave like manner in a direction of natural intestinal contents flow, when said mechanical constriction of the intestine section is released.

Where a valve, in particular the exit valve, comprises a hydraulic compartment, the method of use may involve the step of filling or emptying the compartment with hydraulic fluid in order to change its open-closed-state.

As mentioned before, in a simple embodiment, a conduit may be inserted from outside the patient's body into the intestine, thereby mechanically urging the exit valve to open, when emptying of the reservoir is desired.

### PUMP

Emptying the reservoir by means of a pump may involve the step of acting on a wall of the reservoir by means of the pump so as to reduce the reservoir's volume, thereby emptying the reservoir.

Where the pump and the reservoir are separate from each other and where the pump is furthermore implanted inside the patient's body outside the patient's intestine and comprises at least one electrical stimulation device, the method of use may comprise the steps of electrically stimulating muscle or neural tissue of an intestine section by means of the stimulation device so as to cause at least partial contraction of the intestine section, preferably by applying electric pulses to the intestine section. Again, the step of emptying the reservoir may comprise pumping intestinal contents along the intestine section by, over time, stimulating the different portions of the intestine section in a wave like manner in a direction of natural intestinal contents flow.

The intestinal contents may likewise be pumped along the intestine section by, over time, constricting different portions of an intestine section in a wave like manner in the direction of natural intestinal contents flow by means of a constriction device which is adapted to at least partly constrict the intestine section mechanically. Where the stimulation device is combined with such constriction device, pumping of the intestinal contents along the intestine section by, over time, stimulating the different portions of the intestine section in a wave like manner in a direction of natural intestinal contents flow may be performed, while constriction of the intestine section caused by the constriction device is released at the respective portions.

The pump may be activated by manually operating a subcutaneously arranged actuator from outside the patient's body.

Where a conduit is inserted from outside the patient's body into the intestine, thereby mechanically urging the exit valve to open when emptying the reservoir and where such conduit provides a flow passage to an external collecting device comprising a suction pump, the method of use further comprises the step of emptying the reservoir by means of the suction pump.

### MOTOR

The suction pump may preferably be driven by means of a motor. Also, at least the exit valve is preferably driven between its closed and open positions by means of at least one motor implanted in the patient's body. Similarly, the pump is preferably driven by means of a motor implanted in the patient's body.

In either case, the motor is preferably activated from outside the patient's body by operating a subcutaneously arranged switch.

### ENERGY

As mentioned before, energy may be transmitted from outside the patient's body to at least one implanted energy consuming part of the system, preferably in the form of wireless energy. This may involve the following additional steps:
- transforming the wirelessly transmitted energy into electric energy by means of an energy transforming device,
- storing the transformed energy in an energy storage means, and
- supplying the stored energy from the energy storage means to at least one implanted energy consuming part of the system.

Again, energy may be supplied wirelessly from the storage means to the energy consuming part.

Preferably, at least part of the wirelessly transmitted energy is transformed into electric energy and used for the energy consuming part of the system as said part of the wirelessly transmitted energy is transformed into the electric energy.

### CONTROL

Where a first part of a control unit for controlling at least one energy consuming part of the system is implanted inside the patient's body, the method of use may further comprise the step of using the external second part of the control unit to transmit data to the implanted first part of the control unit. Preferably, the data are transmitted to the implanted first part of the control unit in the same manner as energy is transmitted to the implanted energy consuming part. More particularly, the data are preferably transmitted wirelessly to the implanted first part of the control unit. This may involve a wireless control signal.

For instance, the implanted first part of the control unit can be programmed via the external second part of the control unit. Furthermore, a feedback signal may be transmitted from the implanted first part of the control unit to the external second part of the control unit.

### SENSOR

Where one or more of the afore-mentioned sensors are provided, the method of use may comprise the step of sensing a physical parameter in the patient's body and/or a functional parameter of the system in the patient's body, such as one or more of the following parameters: a pressure within the reservoir, a pressure within the patient's intestine, an expansion of the reservoir, a distension of an intestinal wall of the patient's intestine, a pressure against a part of the system such as the reservoir, a distension of a part of the system such as a wall of the reservoir, an electrical parameter such as voltage, current or energy balance, a position or movement of a movable part of the system, any stimulation parameter in relation to the system.

A signal, such as a sound signal or a visual signal, may be provided when a value for the physical parameter sensed is beyond a predetermined threshold value.

The invention will now be described in more detail in context with some preferred embodiments of the invention as shown in the accompanying drawings.

### Brief description of the drawings

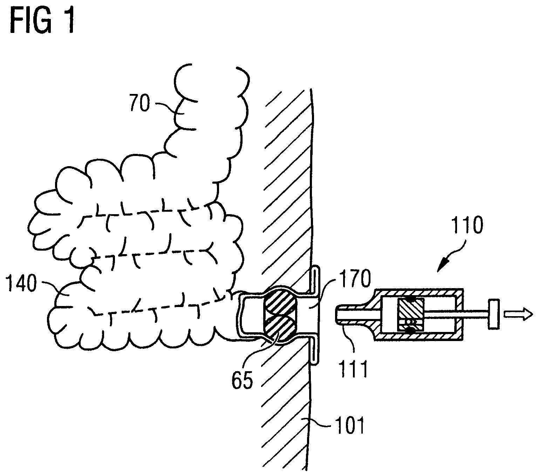

Figure 1 shows a system according to the present invention, wherein the reservoir 140 is formed by a plurality of bent portions of human intestine 70, with laterally adjacent sections thereof being cut open along their mutual contact line and the resulting upper halves and lower halves thereof being interconnected so as to form a reservoir 140. The flow control device consists of one exit valve 65 implanted within the intestine 70, and the intestine 70 exits the patient's abdominal wall 101 through a surgically created stomy 170. An external manually driven suction pump 110 is used
   for emptying the reservoir 140, wherein a conduit 111 on the front end of the pump 110 is inserted from outside the patient's body into the intestine 70, thereby mechanically urging the exit valve 65 to open. Accordingly, the structure of the exit valve 65 is resilient so as to close automatically.

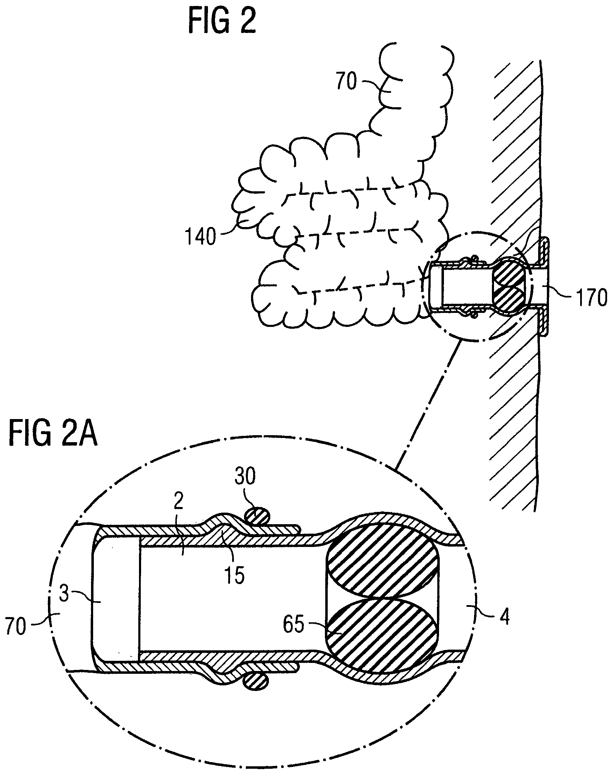

Figure 2 shows a variant to Figure 1. Instead of being implanted inside the patient's intestine 70, the exit valve 65 makes part of an artificial intestine section 2, one end 4 of which forms the stomy opening 170 and the other end 3 of which is affixed by means of a ring-and-bulge connector 15, 30 to the cross-sectional opening of the intestine 70.
Figure 2A shows an enlarged view of the ring-and-bulge connection 15, 30 between the artificial intestine section 2 and the patient's intestine 70.

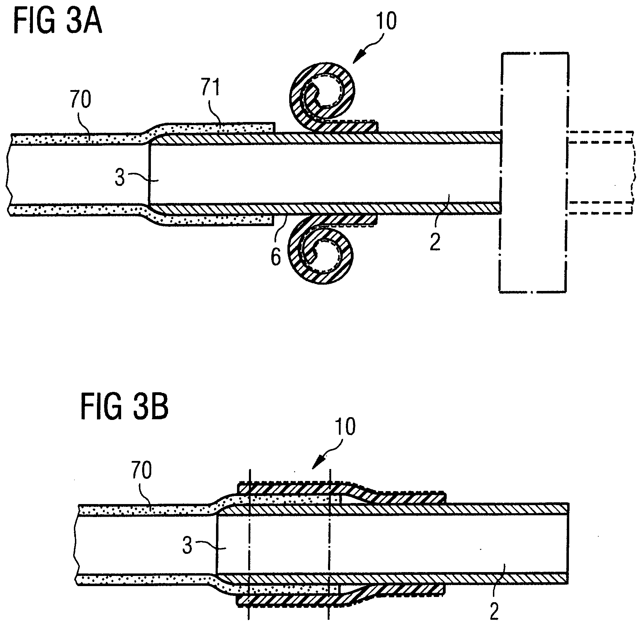

Figure 3A and 3B show an alternative to the ring-and-bulge connection of Figure 2A. Here, the artificial intestine section 2 comprises a conduit and a flexible sleeve 10 which axially extends and closely fits around the outer surface 6 of the conduit 2. The sleeve 10 is rolled upon itself and can be unrolled such that a part 71 of the intestine 70 is located intermediate the sleeve 10 and the conduit 2.

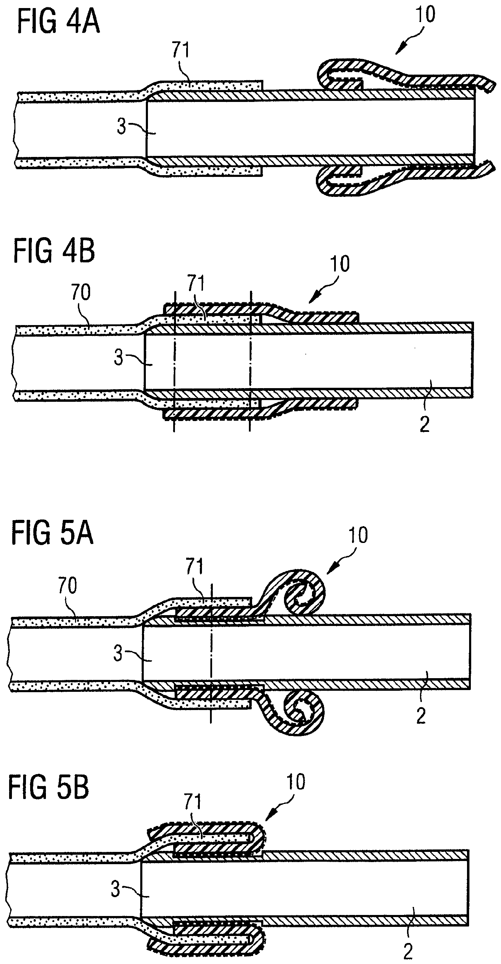

Figures 4A and 4B show an alternative to the connection in Figure 3A and 3B. Instead of unrolling the sleeve 10, it is simply pulled over the intestine 71.
Figure 5A and 5B show another sleeve connection. Here, the sleeve 10 is mounted on the outer surface of the conduit 2 so as be foldable upon itself. By folding the flexible sleeve 10 upon itself, a part 71 of the intestine 70 is located intermediate the folded sleeve 10.

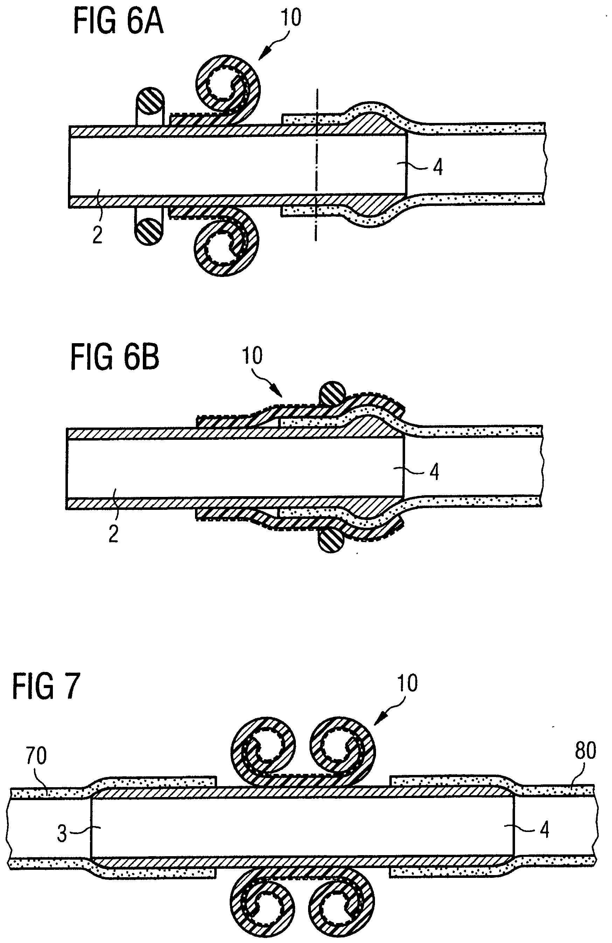

Figure 6A and 6B show a combined connection comprising both the function of the ring-and-bulge connection of Figure 2A and the function of the sleeve connection of Figures 3A and 3B. Combinations of the ring-and-bulge connection with the sleeve connections of Figures 4A, 4B or 5A, 5B are likewise possible.
Figure 7 generally shows that the artificial intestine section may be affixed with both open ends 3, 4 to cross-sectional openings created in the patient's intestine 70, 80, intended for cases where the downstream open end portion of the artificial intestine section is not intended to form a stomy or anus. The artificial intestine section here is shown without any internal components and may comprise a reservoir for intestinal contents, one or more valves, a pump and/or any other flow control device. The connection of the open end portions of the artificial intestine section to the patient's intestine is shown in Figures 7 to be made by sleeve connections, here involving a single sleeve 10.

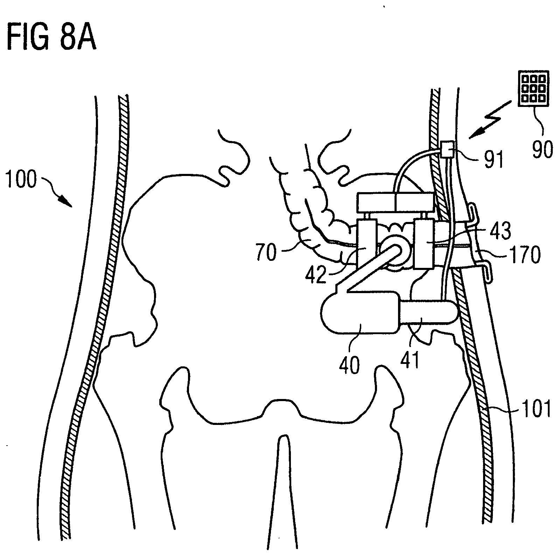

Figure 8A shows an embodiment not forming part of the invention, with an artificial reservoir 40 connected to a lateral opening in the patient's intestine wall. An entry valve 42 and an exit valve 43 are arranged at the patient's intestine upstream and downstream of the reservoir 40. A stomy 170 exiting the patient's abdominal wall 101 has been surgically created from the patient's small or large intestine. The reservoir 40 is mounted with a pump 41 in a common housing and the pump 41 and the entry and exit valves 42, 43 are controlled by means of a control device, of which a part 91 is implanted inside the patient's body 100. Data are transmitted wirelessly between the external part 90 and the implanted part 91 of the control unit. In addition, energy is wirelessly transmitted to an accumulator also implanted in the patient's body and galvanically connected here to the valves and pump.

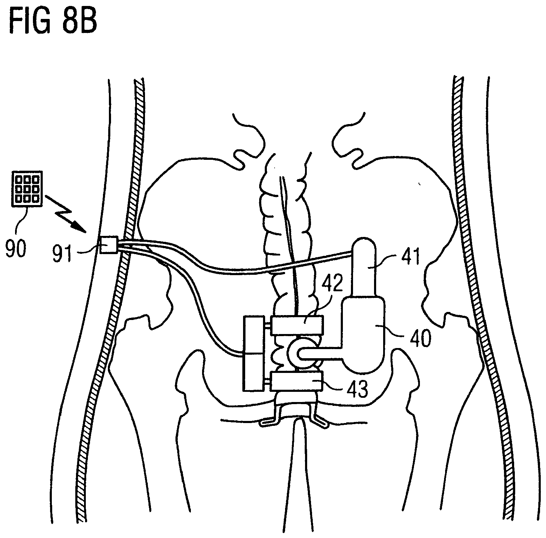

Figure 8B shows the system of Figure 8A not forming part of the invention and connected to the patient's anus rather than to a surgically created stomy.

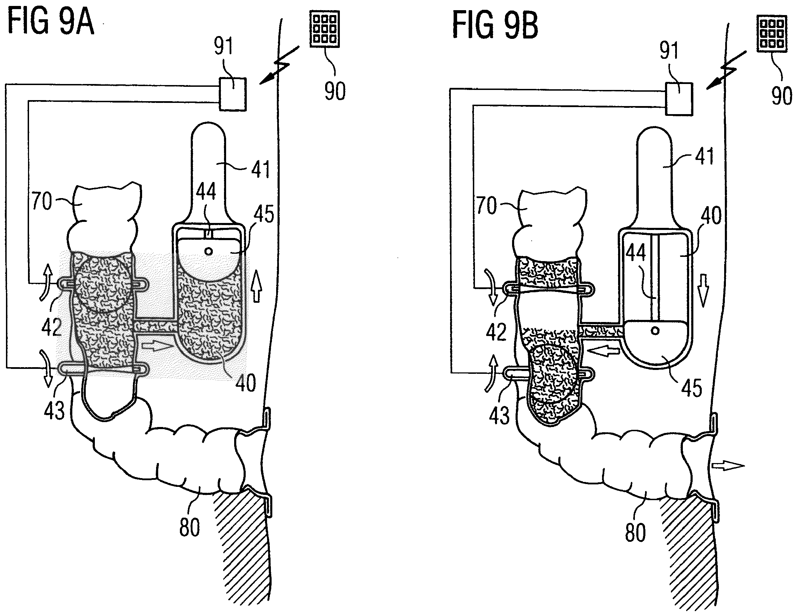

Figures 9A and 9B show a specific embodiment not forming part of the invention, wherein the pump 41 and the reservoir 40 are comprised in a common housing and the pump 41 comprises a moveable piston 44 with a front end 45 of the piston 44 extending into the reservoir 40 such that a volume of the reservoir 40 is reduced upon advancement of the piston 44. The piston 44 is spring loaded so as to urge the piston 44 into a normally retracted position. Furthermore, entry and exit valves 42, 43 are provided in this embodiment, here being realized as flap valves. The flap valves are controlled so that one valve is open while the other one is closed.

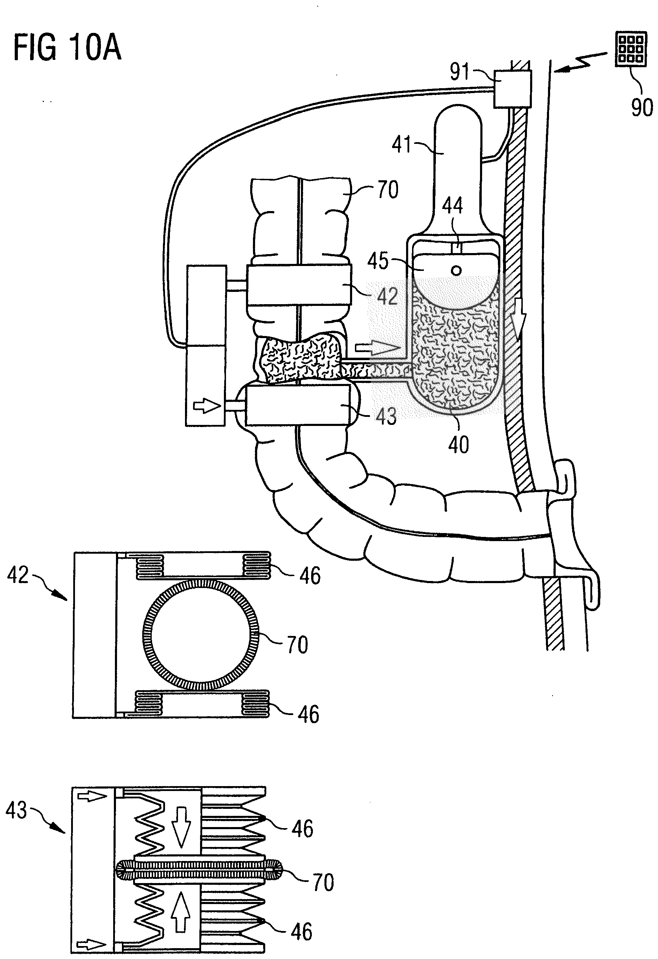

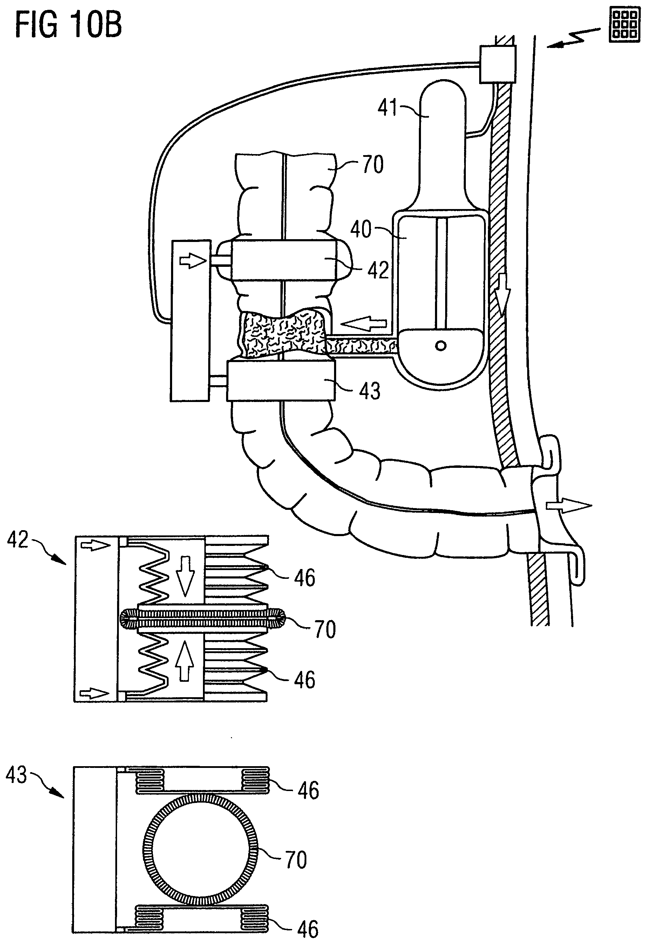

Figures 10A and 10B show a system not forming part of the invention and similar to the one of Figures 9A and 9B. However, here the entry and exit valves 42, 43 comprise bellows 46 acting on the intestine 70 from the outside so as to close the intestine 70 by compression. In Figure 10A the bellows 46 of the exit valve 43 are expanded to compress the intestine 70 at the downstream side of the reservoir 40, whereas in Figure 10B the intestine 70 is closed by means of the bellows 46 of the entry valve 42 upstream of the reservoir 40 so that the reservoir 40 can be emptied by advancing the piston 44 of the pump 41.

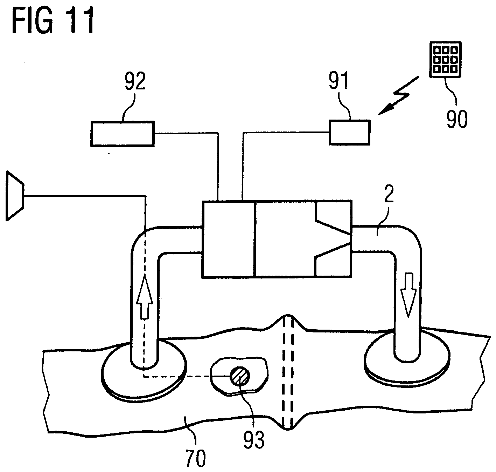

Figure 11 shows an embodiment schematically not forming part of the invention , wherein the artificial intestine section 2 by-passes a section of the patient's intestine70 , the intestine 70 being closed by sewing so as to direct intestinal content towards the artificial intestine section 2. The enlarged area of the artificial intestine section represents any kind of element acting on the intestinal contents within the artificial intestine section, such as a reservoir, one or more valves, a pump or any other flow control device, possibly including a motor, and the like. Furthermore, a battery 92 implantable in the patient's body and preferably rechargeable provides the artificial intestine section 2 with energy. The artificial intestine section 2 is wirelessly controlled and the battery 92, if rechargeable, wirelessly charged. A sensor 93 implanted on or within the intestine 70 delivers data on the physical conditions within the intestine 70 for controlling the artificial intestine section 2.

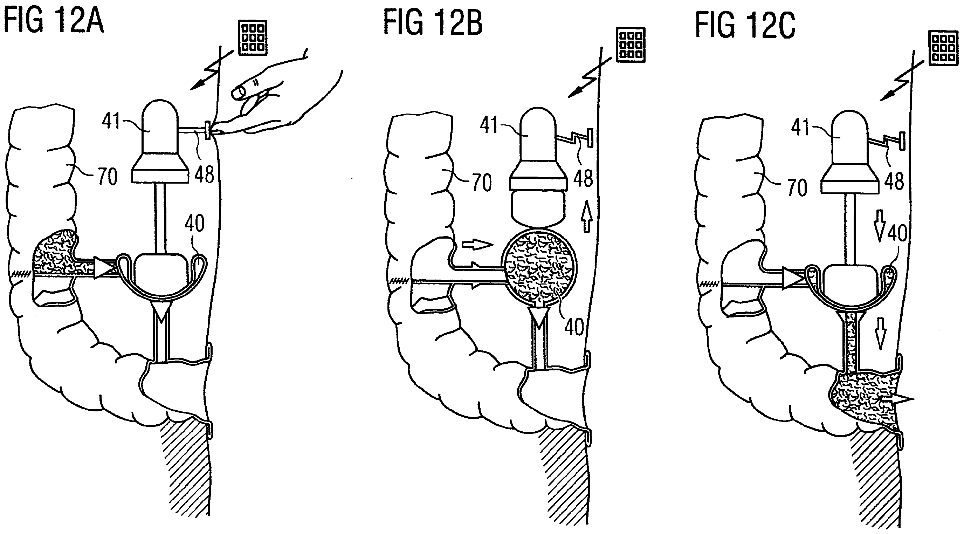

Figures 12A to 12C show a specific embodiment not forming part of the invention, wherein the artificial reservoir 40 by-passes a section of the patient's intestine 70. The reservoir 40 has a flexible wall and a pump 41 implanted in the patient's body separate but in close proximity to the reservoir 40 is used to empty the reservoir 40. The pump 41 is actuated by means of a subcutaneously implanted, manually operable switch 48.

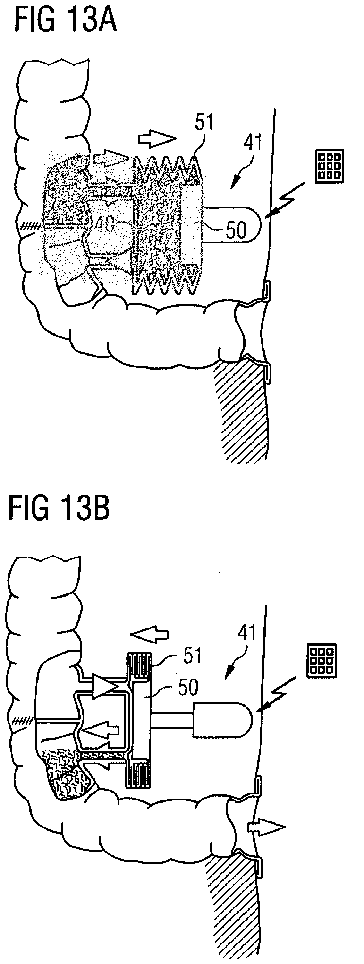

Figures 13A and 13B show a structure similar to the one of Figures 12A to 12C and not forming part of the invention, however, with the pump 41 and the reservoir 40 being fixedly connected to one another. The reservoir 40 is formed by a bellow 51 having an end wall 50 closing the bellow 51 at one end thereof. The end wall 50 makes part of the pump 41 such that a volume of the bellow 51 can be reduced upon advancement of the end wall 50. The bellow 51 is made of a resilient material so as to urge the bellow 51 into a normally extended position.

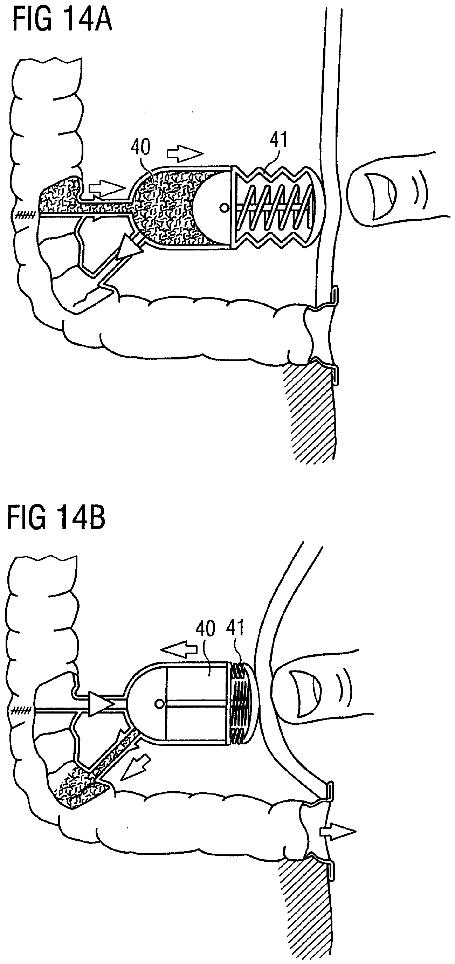

Figures 14A and 14B show a variant to Figures 13A and 13B which does not form part of the invention. Here, the pump 41 and reservoir 40 are integrally combined. The pump 41 is manually operable and subcutaneously mounted so as to be operable from the outside of the patient's body.

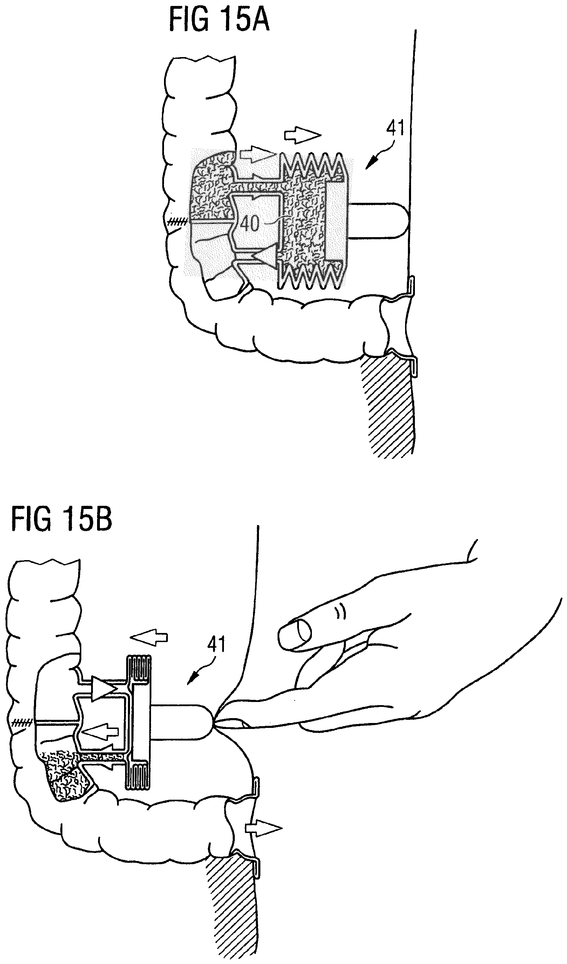

Figures 15A and 15B likewise show a variant to the system shown in Figures 13A and 13B which does not form part of the invention. While in the system of Figures 13A, 13B the pump 41 is automatically driven, such as by an integrated motor, and activated via remote control, the system in Figures 15A and 15B is again manually operable in that the manually operable pump 41 is mounted subcutaneously.

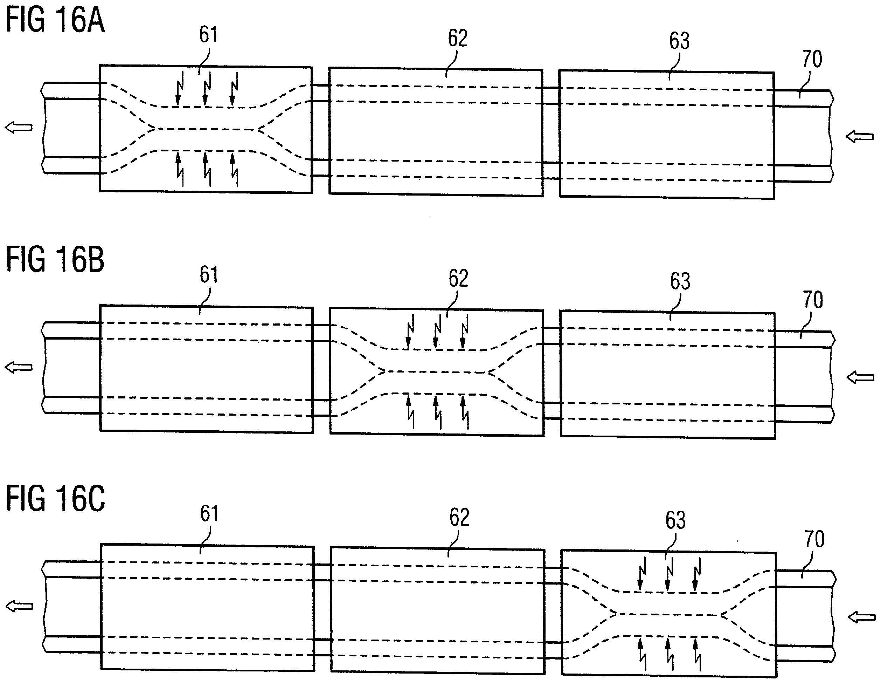

Figures 16A to 16C show a plurality of cooperating valves 61, 62, 63 implanted inside the patient's body and outside the patient's intestine 70. Each of the valves 61, 62, 63 comprises an electrical stimulation device adapted to electrically stimulate muscle or neural tissue of an intestine section so as to cause at least partial contraction of the intestine section. For that purpose, the stimulation device comprises at least one electrode adapted to apply electric pulses to the intestine section. While instead of the three stimulation devices shown, a single stimulation device would be sufficient for opening and closing the intestine, the arrangement of the plurality of stimulation devices is adapted to stimulate different portions of the intestine section over time. The function of the three stimulation devices may also be combined in one integral unit. The direction of natural intestinal contents flow is indicated by arrows. The different portions of the intestine section in a wavelike manner may be made in a direction opposite to the natural intestinal contents flow, as shown in Figures 16A to 16C, so as to close the intestine section. The stimulation in the wavelike manner may also be made in the direction of natural intestinal contents flow to support emptying of the intestine or reservoir.

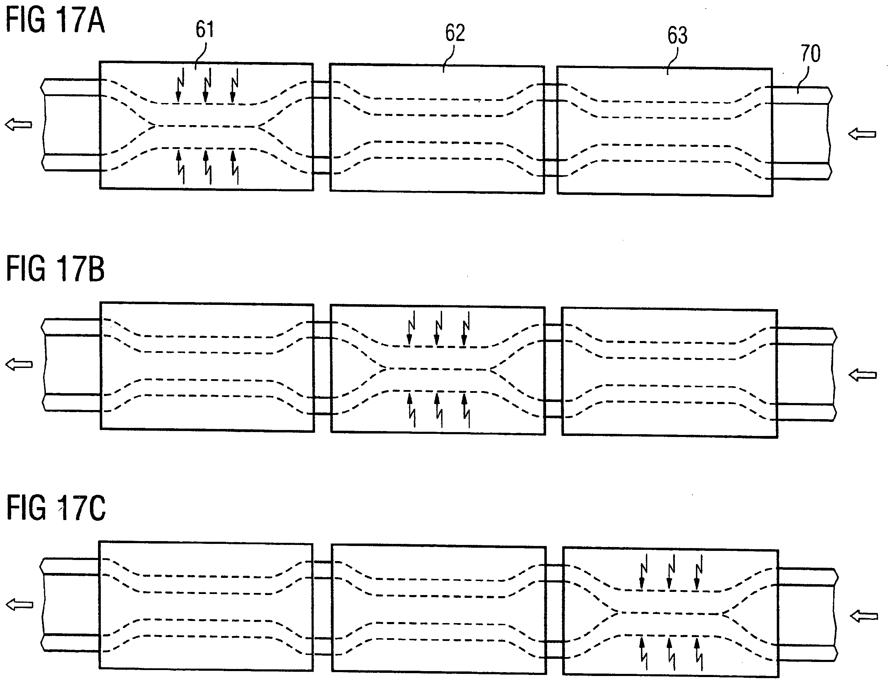

Figures 17A to 17C show the stimulation devices of Figures 16A to 16C in combination with constriction devices, such as the bellow valves described in relation to Figures 10A and 10B, for at least partly constricting the intestine section mechanically. Complete constriction is obtained by additional electrical stimulation of the respective intestine sections. The constriction devices may be released in order to allow intestinal contents to flow through.

Preferred examples are defined in paragraphs 1 to 206.

### INTERNAL RESERVOIR WITH IMPLANTABLE FLOW CONTROL DEVICE

1. A system for treating a patient having a disorder related to the patient's intestine, comprising a reservoir adapted for receiving and temporarily collecting therein intestinal contents and formed by at least one bent portion of human intestine, with laterally adjacent sections thereof being cut open along their mutual contact line and the resulting upper halves and lower halves thereof being interconnected so as to form the reservoir, and further adapted to remain within the patient's body when emptying the reservoir, wherein the system further comprises an artificial flow control device implantable in the patient's body and adapted to control flow of the intestinal contents from the reservoir when implanted in relation thereto, wherein the flow control device comprises a pump for emptying the reservoir and wherein the pump and the reservoir are separate from each other and wherein the pump is adapted to being implanted in the patient's body separate from but in close proximity to the reservoir so as to act on the reservoir from the outside thereof.

### RESERVOIR FORMED FROM INTESTINE OR HUMAN TISSUE

2. The system of para 1, wherein the interconnection is made with staplers.
3. The system of para 1, wherein the reservoir is formed from surgically modified and connected human tissue.

### THROUGH-FLOW ARRANGEMENT OF RESERVOIR

4. The system of para 1, comprising a first passage in flow communication with the reservoir and adapted to being connected to a surgically created first opening of the patient's intestine, said first passage being arranged for transferring intestinal contents to the reservoir, and further comprising a second passage in flow communication with the reservoir, said second passage being arranged for transferring intestinal contents from the reservoir.
5. The system of para 4, wherein the second passage is adapted to being surgically connected to a surgically created stoma.
6. The system of para 4, wherein the second passage is adapted to being surgically connected to the patient's anus or to tissue adjacent the patient's anus.
7. The system of para 4, wherein the second open end portion is adapted to being connected to a portion of a patient's small intestine.
8. The system of para 4, wherein the second open end portion is adapted to being connected to a portion of a patient's large intestine.
9. The system of para 4, wherein the second passage is adapted to being surgically connected to a second surgically created opening of the patient's intestine.

### LATERAL ATTACHMENT

10. The system of para 9, wherein the second passage is adapted to being connected to a lateral opening in a wall of the patient's intestine.
11. The system of any of paras 4 to 10, wherein the first passage is adapted to being connected to a lateral opening in a wall of the patient's intestine.
12. The system of any of paras 4 to 11, wherein at least the first passage is adapted to being bonded to the patient's intestine.
13. The system of any of paras 4 to 12, wherein at least the first passage is adapted to be sewn to the patient's intestine.
14. The system of any of paras 4 to 13, wherein at least the first passage is adapted to be stapled to the patient's intestine.

### VALVE AS PART OF THE FLOW CONTROL DEVICE

15. The system of any of paras 1 to 14, wherein the flow control device comprises at least one valve.

### EXIT VALVE

16. The system of para 16, wherein the at least one valve includes an exit valve preventing intestinal contents flow from the reservoir in its closed position.
17. The system of para 17, wherein the exit valve is a normally closed valve.

### ENTRY VALVE IN ADDITION TO EXIT VALVE

18. The system of any of paras 14 to 17, wherein the flow control device comprises an entry valve allowing intestinal contents to flow towards the reservoir in its open position.
19. The system of para 18, wherein the entry valve is a normally open valve.
20. The system of any of paras 18 to 19, wherein the exit valve and the entry valve are adapted to cooperate such that when one of the two valves is closed, the respective other valve is open, and vice versa.

### VALVE ARRANGEMENT (INTERNAL, INTERSECTING, OUTSIDE)

21. The system of any of paras 15 to 20, wherein at least one of the valve or valves, respectively, is adapted to being permanently implanted inside the patient's intestine.
22. The system of any of paras 15 to 20, wherein at least one of the valve or valves, respectively, has an upstream open end and has a downstream open end in fluid connection with the upstream open end, wherein the upstream open end is adapted to being connected to a surgically created opening of the patient's intestine and the downstream open end is adapted to being connected to either one of a surgically created opening of the patient's intestine and a surgically created stoma.
23. The system of any of paras 15 to 20, wherein at least one of the valve or valves, respectively, has an upstream open end and has a downstream open end in fluid connection with the upstream open end, wherein the upstream open end is adapted to being connected to a surgically created opening of the patient's intestine and the downstream open end is adapted to being surgically connected to either one of the patient's anus or tissue adjacent the patient's anus.
24. The system of any of paras 15 to 20, wherein at least one of the valve or valves, respectively, is adapted to being implanted inside the patient's body outside a section of the patient's intestine and comprises at least one element adapted to act on the intestine section from the outside thereof so as to prevent intestinal contents flow through the intestine section.

### VALVE TYPES

25. The system of any of paras 15 to 24, wherein the at least one valve comprises a central opening which is normally closed by resilient means that can mechanically be urged apart by inserting a conduit through the central opening so as to open the central opening of the valve.
26. The system of any of paras 15 to 25, wherein the at least one valve comprises a compartment with a variable volume adapted to open and close the valve by changing the compartment's volume.
27. The system of para 26, wherein the at least one valve comprises at least one passage for filling and emptying the compartment with hydraulic fluid.
28. The system of any of paras 26 to 27, wherein the compartment has at least one flexible wall defining an opening, the opening being adapted to close upon increase of the compartment's volume.
29. The system of any of paras 21 to 23, wherein the at least one valve is a flap valve.
30. The system of para 29, wherein the flap valve comprises a rotatable disc.
31. The system of para 24, wherein the at least one valve comprises at least one electrical stimulation device adapted to electrically stimulate muscle or neural tissue of an intestine section so as to cause at least partial contraction of the intestine section.
32. The system of para 31, wherein the stimulation device comprises at least one electrode adapted to apply electric pulses to the intestine section.
33. The system of any of paras 31 to 32, wherein the stimulation device is adapted to stimulate different portions of the intestine section over time.
34. The system of para 33, wherein the stimulation device is adapted to stimulate, over time, the different portions of the intestine section in a wave like manner in a direction opposite to natural intestinal contents flow or in any predetermined stimulation pattern.
36. The system of any of paras 31 to 34, wherein the at least one valve comprises a constriction device for at least partly constricting the intestine section mechanically.
37. The system of para 35, wherein the stimulation device is combined with the constriction device so that the stimulation device and the constriction device act on the same intestine section.
38. The system of para 36, wherein the constriction device in its normal condition constricts the intestine section only partly.
38. The system of any of paras 35 to 37, wherein the stimulation device and the constriction device are adapted to act on the same intestine section so as to keep the intestine section closed.
39. The system of any of paras 35 to 38, wherein the stimulation device is adapted to pump intestinal contents along the intestine section by, over time, stimulating different portions of the intestine section in a wave like manner, when constriction of the intestine section caused by the constriction device is released.
40. The system of para 39, wherein the stimulation device is adapted to pump intestinal contents along the intestine section in a direction of natural intestinal contents flow.

### PUMP AS PART OF THE IMPLANTABLE FLOW CONTROL DEVICE

41. The system of para 1, wherein the pump is adapted for emptying the reservoir by squeezing the reservoir.
42. The system of para 1, wherein the reservoir has a flexible wall and the pump comprises a movable piston, with a front end of the piston adapted to act on the flexible wall of the reservoir from the outside thereof upon advancement of the piston.
43. The system of para 42, wherein the piston is spring loaded so as to urge the piston into a normally retracted position.
44. The system of para 1, wherein the pump is adapted to being implanted in the patient's body outside the patient's intestine, wherein the pump comprises at least one electrical stimulation device adapted to electrically stimulate muscle or neural tissue of an intestine section so as to cause at least partial contraction of the intestine section.
45. The system of para 44, wherein the stimulation device comprises at least one electrode adapted to apply electric pulses to the intestine section.
46. The system of any of paras 55 to 45, wherein the stimulation device is adapted to stimulate different portions of the intestine section over time and to pump intestinal contents along the intestine section by, over time, stimulating the different portions of the intestine section in a wave like manner in a direction of natural intestinal contents flow.
47. The system of any of paras 1 to 46, wherein the pump comprises a constriction device for at least partly constricting the intestine section mechanically.
48. The system of para 47, wherein the constriction device is adapted to pump intestinal contents along the intestine section by, over time, constricting different portions of an intestine section in a wave like manner in a direction of natural intestinal contents flow.
49. The system of para 46, wherein the stimulation device is combined with a constriction device for at least partly constricting the intestine section mechanically so as to pump intestinal contents along the intestine section by, over time, stimulating the different portions of the intestine section in a wave like manner in a direction of natural intestinal contents flow, when constriction of the intestine section caused by the constriction device is released at the respective portions.
50. The system of any of paras 1 to 43, wherein the pump is manually drivable and comprises an actuator for manually driving the pump, the actuator being arranged for subcutaneous implantation so as to be operable from outside the patient's body.

### MOTOR

51. The system of any of paras 1 to 50, comprising at least one motor arranged for automatically driving one or more elements of the flow control device.
52. The system of para 51, including any of paras 15 to 40, wherein the at least one motor is arranged for driving at least one of the valve or valves, respectively, between its closed and open position.
53. The system of any of paras 51 to 52, including any of paras 40 to 49, wherein the at least one motor is arranged for driving the pump.
54. The system of any of paras 51 to 53, comprising a manually operable switch for activating the at least one motor, the switch being arranged for subcutaneous implantation so as to be operable from outside the patient's body.
55. The system of any of paras 51 to 54, wherein the motor is arranged to be driven by electric or electromagnetic energy.

### ENERGY SOURCE

56. The system of any of paras 1 to 55, comprising an energy source for supplying energy directly or indirectly to at least one energy consuming part of the system.
57. The system of para 56, wherein said energy source includes a battery as an energy storage means.
58. The system of para 56, wherein said energy source includes an accumulator as an energy storage means.
59. The system of para 58, wherein the accumulator comprises one or more of a rechargeable battery and a capacitor.
60. The system of any of paras 57 to 59, wherein the energy storage means is adapted for being implanted inside the patient's body.
61. The system of any of paras 57 to 60, wherein the energy storage means supplies energy for at least one energy consuming part of the system.

### WIRELESS ENERGY TRANSMISSION

62. The system of para 56, wherein the energy source comprises a wireless energy transmitter adapted to wirelessly transmit energy from outside the patient's body to the at least one energy consuming part.
63. The system of any of paras 57 to 61, wherein the energy source comprises a wireless energy transmitter adapted to wirelessly transmit energy from outside the patient's body to the energy storage means.

### ENERGY TRANSMISSION FEEDBACK

64. The system of para 63, comprising a feedback subsystem adapted to wirelessly send feedback information related to the energy to be stored in the accumulator from inside the human body to the outside thereof, wherein the system is adapted to use the feedback information for adjusting the amount of wireless energy transmitted by the energy transmitter.
65. The system of para 64, wherein the feedback information is related to an energy balance which is defined as the balance between wireless energy received inside the human body and energy consumed by the at least one energy consuming part.
66. The system of para 65, wherein the feedback information is related to an energy balance which is defined as the balance between a rate of wireless energy received inside the human body and a rate of energy consumed by the at least one energy consuming part.
67. The system of any of paras 62 to 66, comprising a wireless energy transmitter adapted to wirelessly transmit energy from the energy storage means to the at least one energy consuming part.
68. The system of any of paras 62 or 67, wherein the energy consuming part is adapted to directly transform the wirelessly transmitted energy into kinetic energy.
69. The system of any of paras 62 to 67, comprising an implantable energy transforming device for transforming wireless energy into electric energy.
70. The system of para 69, wherein the energy consuming part is driven with the electric energy, as said energy transforming device transforms the wireless energy into the electric energy.

### GALVANIC ENERGY TRANSMISSION

71. The system of any of paras 56 to 61, further comprising galvanic coupling elements between the energy source and the motor for transmitting energy to the motor in contacting fashion.

### CONTROL UNIT

72. The system of any of paras 1 to 71, comprising a control unit adapted to directly or indirectly control one or more elements of the system.
73. The system of para 72, including para 16, wherein the control unit is adapted to control opening of the exit valve.
74. The system of any of paras 72 to 73, including para 18, wherein the control unit is adapted to control closing of the entry valve.
75. The system of para 74, wherein the control unit is adapted to control opening of the exit valve and closing of the entry valve such that when one of the two valves is closed, the respective other valve is open, and vice versa.
76. The system of any of paras 72 to 75, including para 53, wherein the control unit is adapted to directly or indirectly control actuation of the pump.
77. The system of any of paras 72 to 76, wherein the control unit is operable by the patient.
78. The system of any of paras 72 to 77, wherein at least part of the control unit is implantable in the patient's body.
79. The system of para 78, comprising a manually operable switch for activating the control unit, the switch being arranged for subcutaneous implantation so as to be operable from outside the patient's body.
80. The system of any of paras 72 to 79, wherein the control unit comprises a first part adapted for implantation in the patient's body and a second part adapted to cooperate with the first part from outside the patient's body.
81. The system of para 80, including para 61, wherein the control unit is adapted to transmit data from the second part of the control unit to the implantable first part of the control unit in the same manner as energy is transmitted to the at least one energy consuming part.
82. The system any of paras 80 to 81, wherein the second part of the control unit is adapted to wirelessly transmit a control signal to the implantable first part of the control unit for controlling the at least one energy consuming part from outside the patient's body.
83. The system of any of paras 80 to 82, wherein the implantable first part of the control unit is programmable via the second part of the control unit.
84. The system of any of paras 80 to 83, wherein the implantable first part of the control unit is adapted to transmit a feedback signal to the second part of the control unit.

### SENSOR

85. The system of any of paras 1 to 84, comprising a physical parameter sensor adapted to directly or indirectly sense a physical parameter of the patient.
86. The system of para 85, wherein the physical parameter sensor is adapted to sense at least one of the following physical parameters of the patient: a pressure within the reservoir, a pressure within the patient's intestine, an expansion of the reservoir, a distension of an intestinal wall of the patient's intestine, a movement of the intestinal wall.
87. The system of any of paras 1 to 86, comprising a functional parameter sensor adapted to directly or indirectly sense a functional parameter of the system.
88. The system of para 87, wherein the functional parameter sensor is adapted to sense at least one of the following functional parameters of the system: a pressure against a part of the system such as the reservoir, a distension of a part of the system such as a wall of the reservoir, an electrical parameter such as voltage, current or energy balance, a position or movement of a movable part of the system.
89. The system of any of paras 85 to 88, comprising an indicator coupled to the sensor, the indicator being adapted to provide a signal when the sensor senses a value for the parameter beyond a predetermined threshold value.
90. The system of para 89, wherein the signal comprises at least one of the following types of signals: a sound signal, a visual signal.

### INTESTINAL CONTENTS COLLECTING DEVICE (WITH "EXTERNAL" PUMP)

91. The system of any of paras 1 to 90, further comprising an intestinal contents collecting device to be temporarily applied from outside the patient's body.
92. The system of para 91, including para 16, wherein the collecting device comprises a front open end adapted to be applied towards said exit valve so as to provide a flow passage from the exit valve towards the collecting device.
93. The system of para 92, wherein the collecting device front open end is adapted to be applied to said exit valve so as to open the valve and thereby provide the flow passage towards the collecting device.
94. The system of para 93, including para 25, wherein said front open end is adapted to be inserted through the central opening of said exit valve so as to urge apart the resilient means normally closing the central opening.
95. The system of any of paras 91 to 94, wherein the collecting device comprises a suction pump.
96. The system of para 95, wherein the suction pump comprises a piston-cylinder-arrangement.
97. The system of any of paras 95 to 96, wherein the suction pump is adapted to be driven manually.
98. The system of any of paras 95 to 97, further comprising a motor connected to the suction pump for driving the pump automatically.
99. The system of any of paras 95 to 98, including any of paras 1 to 55, wherein the suction pump is provided as a back-up pump for a situation where the pump of the flow control device is out of operation.

## Claims

1. A system (100) for treating a patient having a disorder related to the patient's intestine (70) and further having a reservoir (40) provided for receiving and temporarily collecting therein intestinal contents and formed by at least one bent portion of human intestine, with laterally adjacent sections thereof being cut open along their mutual contact line and the resulting upper halves and lower halves thereof being interconnected so as to form the reservoir (40), and further adapted to remain within the patient's body when emptying the reservoir (40), said system (100) comprising: an artificial flow control device implantable in the patient's body and adapted to control flow of the intestinal contents from the reservoir (40) when implanted in relation thereto, wherein the flow control device comprises a pump (41) for emptying the reservoir (40) and wherein the pump (41) and the reservoir (40) are separate from each other and wherein the pump (41) is adapted to being implanted in the patient's body separate from but in close proximity to the reservoir (40) so as to act on the reservoir (40) from the outside thereof.

2. The system of claim 1, wherein the pump (41) is adapted for emptying the reservoir (40) by squeezing the reservoir (40).

3. The system of claim 1, wherein the reservoir (40) has a flexible wall and the pump (41) comprises a movable piston (44), with a front end (45) of the piston (44) adapted to act on the flexible wall of the reservoir (40) from the outside thereof upon advancement of the piston (44).

4. The system of any of claim 3, wherein the piston (44) is spring loaded so as to urge the piston (44) into a normally retracted position.

5. The system of claim 1, wherein the pump (41) is adapted to being implanted in the patient's body outside the patient's intestine (70), wherein the pump (41) comprises at least one electrical stimulation device adapted to electrically stimulate muscle or neural tissue of an intestine section so as to cause at least partial contraction of the intestine section.

6. The system of claim 5, wherein the stimulation device comprises at least one electrode adapted to apply electric pulses to the intestine section.

7. The system of claim 5 or 6, wherein the stimulation device is adapted to stimulate different portions of the intestine section over time and to pump intestinal contents along the intestine section by, over time, stimulating the different portions of the intestine section in a wave like manner in a direction of natural intestinal contents flow.

8. The system of any of claims 1 to 7, wherein the pump (41) comprises a constriction device for at least partly constricting the intestine section mechanically.

9. The system of claims 8, wherein the constriction device is adapted to pump intestinal contents along the intestine section by, over time, constricting different portions of an intestine section in a wave like manner in a direction of natural intestinal contents flow.

10. The system of claim 7, wherein the stimulation device is combined with a constriction device for at least partly constricting the intestine section mechanically so as to pump intestinal contents along the intestine section by, over time, stimulating the different portions of the intestine section in a wave like manner in a direction of natural intestinal contents flow, when constriction of the intestine section caused by the constriction device is released at the respective portions.

11. The system of any of claims 1 to 4, wherein the pump (41) is manually drivable and comprises an actuator for manually driving the pump, the actuator being arranged for subcutaneous implantation so as to be operable from outside the patient's body.

12. The system of any of claims 1 to 11, comprising at least one motor arranged for automatically driving one or more elements of the flow control device.

13. The system of claim 12, including any of claims 1 to 10, wherein the at least one motor is arranged for driving the pump (41).

14. The system of claim 12 or 13, comprising a manually operable switch (48) for activating the at least one motor, the switch (48) being arranged for subcutaneous implantation so as to be operable from outside the patient's body.

15. The system of any of claims 12 to 14, wherein the motor is arranged to be driven by electric or electromagnetic energy.

## Patentansprüche

1. System (100) zum Behandeln eines Patienten, der eine Störung in Bezug auf den Darm (70) des Patienten hat und des Weiteren ein Reservoir (40) hat, das zum Aufnehmen und temporären Sammeln von Darminhalten darin bereitgestellt ist und durch zumindest einen gebogenen Teil menschlichen Darms gebildet ist, wobei seitlich aneinander grenzende Abschnitte davon entlang ihrer gegenseitigen Kontaktlinie aufgeschnitten sind und die resultierenden oberen Hälften und unteren Hälften davon miteinander verbunden sind, um das Reservoir (40) zu bilden, und das des Weiteren angepasst ist, innerhalb des Körpers des Patienten zu verbleiben, wenn das Reservoir (40) geleert wird, wobei das System (100) umfasst: eine künstliche Flusssteuervorrichtung, die in den Körper des Patienten implantierbar ist und angepasst ist, einen Fluss der Darminhalte von dem Reservoir (40) zu steuern, wenn sie in Relation dazu implantiert ist, wobei die Flusssteuervorrichtung eine Pumpe (41) zum Leeren des Reservoirs (40) umfasst und wobei die Pumpe (41) und das Reservoir (40) separat voneinander sind und wobei die Pumpe (41) angepasst ist, in den Körper des Patienten separat aber in naher Nähe zum Reservoir (40) implantiert zu werden, um auf das Reservoir (40) von außerhalb davon zu wirken.

2. System nach Anspruch 1, wobei die Pumpe (41) zum Leeren des Reservoirs (40) durch Zusammendrücken des Reservoirs (40) angepasst ist.

3. System nach Anspruch 1, wobei das Reservoir (40) eine flexible Wand aufweist und die Pumpe (41) einen beweglichen Kolben (44) umfasst, wobei ein vorderes Ende (45) des Kolbens (44) angepasst ist, auf die flexible Wand des Reservoirs (40) von außerhalb davon beim Vorschieben des Kolbens (44) zu wirken.

4. System nach Anspruch 3, wobei der Kolben (44) federbelastet ist, um den Kolben (44) in eine normalerweise zurückgezogene Position zu drängen.

5. System nach Anspruch 1, wobei die Pumpe (41) angepasst ist, in den Körper des Patienten außerhalb des Darms (70) des Patienten implantiert zu werden, wobei die Pumpe (41) zumindest eine elektrische Stimulationsvorrichtung umfasst, die angepasst ist, Muskel- oder Nervengewebe eines Darmabschnitts elektrisch zu stimulieren, um eine zumindest teilweise Kontraktion des Darmabschnitts zu bewirken.

6. System nach Anspruch 5, wobei die Stimulationsvorrichtung zumindest eine Elektrode umfasst, die angepasst ist, elektrische Impulse auf den Darmabschnitt aufzubringen.

7. System nach Anspruch 5 oder 6, wobei die Stimulationsvorrichtung angepasst ist, im Verlauf der Zeit verschiedene Teile des Darmabschnitts zu stimulieren und Darminhalte entlang des Darmabschnitts zu pumpen, durch im Verlauf der Zeit Stimulieren der verschiedenen Teile des Darmabschnitts auf eine wellenartige Weise in einer Richtung eines natürlichen Darminhaltflusses.

8. System nach einem der Ansprüche 1 bis 7, wobei die Pumpe (41) eine Verengungsvorrichtung zum zumindest teilweisen Verengen des Darmabschnitts auf mechanische Weise umfasst.

9. System nach Anspruch 8, wobei die Verengungsvorrichtung angepasst ist, Darminhalte entlang des Darmabschnitts zu pumpen, durch im Verlauf der Zeit Verengen von verschiedenen Teilen eines Darmabschnitts auf eine wellenartige Weise in einer Richtung eines natürlichen Darminhaltflusses.

10. System nach Anspruch 7, wobei die Stimulationsvorrichtung mit einer Verengungsvorrichtung zum zumindest teilweise Verengen des Darmabschnitts auf mechanische Weise kombiniert ist, um Darminhalte entlang des Darmabschnitts zu pumpen, durch im Verlauf der Zeit Stimulieren der verschiedenen Teile des Darmabschnitts auf eine wellenartige Weise in einer Richtung eines natürlichen Darminhaltflusses, wenn eine durch die Verengungsvorrichtung bewirkte Verengung des Darmabschnitts an den entsprechenden Teilen gelöst wird.

11. System nach einem der Ansprüche 1 bis 4, wobei die Pumpe (41) manuell antreibbar ist und einen Aktuator zum manuellen Antreiben der Pumpe umfasst, wobei der Aktuator zur subkutanen Implantation eingerichtet ist, um von außerhalb des Körpers des Patienten bedienbar zu sein.

12. System nach einem der Ansprüche 1 bis 11, umfassend zumindest einen Motor, der zum automatischen Antreiben eines oder mehrerer Elemente der Flusssteuervorrichtung eingerichtet ist.

13. System nach Anspruch 12, einschließlich einem der Ansprüche 1 bis 10, wobei der zumindest eine Motor zum Antreiben der Pumpe (41) eingerichtet ist.

14. System nach Anspruch 12 oder 13, umfassend einen manuell bedienbaren Schalter (48) zum Aktivieren des zumindest einen Motors, wobei der Schalter (48) zur subkutanen Implantation eingerichtet ist, um von außerhalb des Körpers des Patienten bedienbar zu sein.

15. System nach einem der Ansprüche 12 bis 14, wobei der Motor eingerichtet ist, durch elektrische oder elektromagnetische Energie angetrieben zu werden.

## Revendications

1. Système (100) pour traiter un patient atteint d'un trouble lié à l'intestin (70) du patient et ayant en outre un réservoir (40) prévu pour y recevoir et collecter temporairement des contenus intestinaux et formé par au moins une partie pliée de l'intestin humain, avec ses sections latéralement adjacentes qui sont ouvertes par découpe le long de leur ligne de contact mutuelle et leurs moitiés supérieures et leurs moitiés inférieures résultantes étant interconnectées afin de former le réservoir (40) et en outre étant adaptées pour rester dans le corps du patient lors du vidage du réservoir (40), ledit système (100) comprenant : un dispositif de contrôle d'écoulement artificiel implantable dans le corps du patient et adapté pour contrôler l'écoulement des contenus intestinaux à partir du réservoir (40) lorsqu'ils sont implantés par rapport à ce dernier, dans lequel le dispositif de contrôle d'écoulement comprend une pompe (41) pour vider le réservoir (40) et dans lequel la pompe (41) et le réservoir (40) sont séparés l'un de l'autre et dans lequel la pompe (41) est adaptée pour être implantée dans le corps du patient séparée mais à proximité immédiate du réservoir (40) afin d'agir sur le réservoir (40) à partir de son extérieur.

2. Système selon la revendication 1, dans lequel la pompe (41) est adaptée pour vider le réservoir (40) en appuyant sur le réservoir (40).

3. Système selon la revendication 1, dans lequel le réservoir (40) a une paroi flexible et la pompe (41) comprend un piston mobile (44), avec une extrémité avant (45) du piston (44) adaptée pour agir sur la paroi flexible du réservoir (40) depuis son extérieur suite à l'avancement du piston (44).

4. Système selon la revendication 3, dans lequel le piston (44) est chargé par ressort afin de pousser le piston (44) dans une position normalement rétractée.

5. Système selon la revendication 1, dans lequel la pompe (41) est adaptée pour être implantée dans le corps du patient à l'extérieur de l'intestin (70) du patient, dans lequel la pompe (41) comprend au moins un dispositif de stimulation électrique adapté pour stimuler électriquement le tissu musculaire ou le tissu neural d'une section intestinale afin de provoquer au moins une contraction partielle de la section intestinale.

6. Système selon la revendication 5, dans lequel le dispositif de stimulation comprend au moins une électrode adaptée pour appliquer des impulsions électriques sur la section intestinale.

7. Système selon la revendication 5 ou 6, dans lequel le dispositif de stimulation est adapté pour stimuler différentes parties de la section intestinale au fil du temps et pour pomper le contenu intestinal le long de la section intestinale, en stimulant, au fil du temps, les différentes parties de la section intestinale à la manière d'une onde dans une direction d'écoulement naturel de contenu intestinal.

8. Système selon l'une quelconque des revendications 1 à 7, dans lequel la pompe (41) comprend un dispositif de constriction pour resserrer au moins partiellement mécaniquement la section intestinale.

9. Système selon la revendication 8, dans lequel le dispositif de constriction est adapté pour pomper le contenu intestinal le long de la section intestinale, au fil du temps, en resserrant différentes parties d'une section intestinale à la manière d'une onde dans une direction d'écoulement naturel de contenu intestinal.

10. Système selon la revendication 7, dans lequel le dispositif de stimulation est combiné avec un dispositif de constriction pour resserrer au moins partiellement mécaniquement la section intestinale afin de pomper le contenu intestinal le long de la section intestinale, en stimulant, au fil du temps, les différentes parties de la section intestinale à la manière d'une onde dans une direction d'écoulement naturel de contenu intestinal, lorsque la constriction de la section intestinale provoquée par le dispositif de constriction est relâchée au niveau des parties respectives.

11. Système selon l'une quelconque des revendications 1 à 4, dans lequel la pompe (41) peut être entraînée manuellement et comprend un actionneur pour entraîner manuellement la pompe, l'actionneur étant agencé pour l'implantation sous-cutanée afin de pouvoir être actionné depuis l'extérieur du corps du patient.

12. Système selon l'une quelconque des revendications 1 à 11, comprenant au moins un moteur agencé pour entraîner automatiquement un ou plusieurs éléments du dispositif de contrôle d'écoulement.

13. Système selon la revendication 12, comprenant l'une quelconque des revendications 1 à 10, dans lequel le au moins un moteur est agencé pour entraîner la pompe (41).

14. Système selon la revendication 12 ou 13, comprenant un commutateur pouvant être actionné manuellement (48) pour activer le au moins un moteur, le commutateur (48) étant agencé pour l'implantation sous-cutanée afin de pouvoir être actionné depuis l'extérieur du corps du patient.

15. Système selon l'une quelconque des revendications 12 à 14, dans lequel le moteur est agencé pour être entraîné par l'énergie électrique ou électromagnétique.
